# EUROPEAN PATENT SPECIFICATION

(11) **EP 2 767 282 B1**
(45) Date of publication and mention of the grant of the patent: **15.04.2020**
(21) Application number: 12839969.8
(22) Date of filing: 09.10.2012
(51) Int. Cl.: A61K 9/02, A61K 9/20, A61K 31/417, A61K 31/43, A61K 31/5383, A61K 31/546, A61K 31/7052, A61K 31/7048, A61K 45/06, A61P 11/00

(54) **USE OF GLUTARYL HISTAMINE TO TREAT RESPIRATORY TRACT INFECTIONS**
VERWENDUNG VON GLUTARYLHISTAMIN ZUR BEHANDLUNG VON ATEMWEGSINFEKTIONEN
UTILISATION DE LA GLUTARYLHISTAMINE POUR TRAITER LES MALADIES DES VOIES RESPIRATOIRES

(30) Priority: 11.10.2011 RU 2011141288
(43) Date of publication of application: 20.08.2014
(73) Proprietor: Ltd. "Valenta-Intellekt", Moscow 119530 (RU); Nebolsin, Vladimir Evgenievich, Moskovskaya Obl., 143581 (RU)
(72) Inventor: NEBOLSIN, Vladimir Evgenievich, Moskovskaya Obl., 143581 (RU); KOLOBUKHINA, Lyudmila Vasilievna, Moscow, 127204 (RU)
(74) Representative: Hoffmann Eitle
(86) International application number: PCT/RU2012/000821
(87) International publication number: WO 2013/055258

(56) References cited:
- WO-A1-2010/134851
- DATABASE BIOSIS [Online] BIOSCIENCES INFORMATION SERVICE, PHILADELPHIA, PA, US; 2011, KOLOBUKHINA L V ET AL: "The efficacy of Ingavirin in the combined treatment of ARVI complicated by tonsilitis", XP002735031, Database accession no. PREV201200299000 & VESTNIK OTORINOLARINGOLOGII, no. 6, 2011, pages 91-95, ISSN: 0042-4668
- DATABASE BIOSIS [Online] BIOSCIENCES INFORMATION SERVICE, PHILADELPHIA, PA, US; September 2011 (2011-09), KOLOBUKHINA L V ET AL: "PANDEMIC FLU IN RUSSIA: SPECIAL FEATURES OF A CLINICAL COURSE AND THE ABSENCE OF EARLY ETIOTROPIC THERAPY AS A RISK FACTOR OF SEVERE FORMS OF THE DISEASE", XP002735032, Database accession no. PREV201200054001 & TERAPEVTICHESKII ARKHIV, vol. 83, no. 9, 2011, pages 48-53, ISSN: 0040-3660
- DATABASE BIOSIS [Online] BIOSCIENCES INFORMATION SERVICE, PHILADELPHIA, PA, US; May 2010 (2010-05), LENEVA I A ET AL: "Study of the antiviral activity of Russian anti-influenza agents in cell culture and animal models", XP002735033, Database accession no. PREV201000474369 & VOPROSY VIRUSOLOGII, vol. 55, no. 3, May 2010 (2010-05), pages 19-27, ISSN: 0507-4088
- DATABASE BIOSIS [Online] BIOSCIENCES INFORMATION SERVICE, PHILADELPHIA, PA, US; July 2004 (2004-07), KOVALEVA V L ET AL: "The effect of a potential drug ingamine on a model of noninfectious pneumonia", XP002735034, Database accession no. PREV200500008302 & KOVALEVA V L ET AL: "The effect of a potential drug ingamine on a model of noninfectious pneumonia", EKSPERIMENTAL'NAYA I KLINICHESKAYA FARMAKOLOGIYA, vol. 67, no. 4, July 2004 (2004-07), pages 30-34, ISSN: 0869-2092
- MURRAY N. W.: 'Current and future clinical applications of interferon-gamma in host antimicrobial defense' INTENSIVE CARE MEDICINE vol. 22, no. ISS.4, October 1996, pages S456 - S461, XP055149625
- NONIKOV V.E.: 'Pnevmonii: vybor antibakterialnoi terapii.' PULMONOLOGIIA no. 1, 2010, pages 7 - 11, XP055160086 Retrieved from the Internet: <URL:http://www.consilium-medicum.com/artic le/19963> [retrieved on 2013-02-07]

## Description

### Field of the art

The invention relates to medicine, in particular, to glutaryl histamine and pharmaceutically acceptable salts thereof for use in the treatment of respiratory tract diseases.

### Background

Respiratory tract bacterial infections are widely disseminated and frequently require antibacterial therapy (Antimicrobial Treatment Guidelines for Acute Bacterial Rhinosinusitis. Otolaryngol Head Neck Surg 2004; 130(suppl): S1-45).

Moreover, in recent years, the world has witnessed a significant increase in resistance of respiratory tract pathogens to antibacterial agents, such as beta-lactam antibiotics which are widely used in the treatment of respiratory tract infections. There is a need in research to be able to find ways to overcome such resistance, in particular, one of them could be the development of new medicaments potentiating the action of antimicrobial agents, which makes it possible to restrict the spread of antibiotic-resistant respiratory tract pathogens and to increase the effectiveness of treatment.

Thus, there is a need for effective medicaments for the treatment of respiratory tract diseases which, in a preferred embodiment, potentiate the action of antibacterial agents.

The group of N-acyl derivatives of biogenic amines, including the compound according to the invention (glutaryl histamine), has been disclosed in patent RU 2141483.

Furthermore, the use of ingavirin (glutaryl histamine) in the treatment of flu caused by pandemic virus of flu (H1N1) is described in the prior art (L. V. Kolobukhina et al., "Pandemic flu in Russia: special features of a clinical course and the absence of early etiotropic therapy as a risk factor of severe formsof the disease", Terapevticheskii Arkhiv, 2011, Vol. 83, No. 9, p. 48-53).

Moreover, there has been described the use of ingamine (glutaryl histamine) for the treatment of non-infectious pneumonia in the stage of chronic inflammation (L. V. Kovaleva et al., "The effect of a potential drug ingamine on a model of noninfectious pneumonia", Eksperimental'naya i Klinicheskaya Farmakologiya, 2004, Vol. 67, No. 4, p. 30-34).

The authors of the present invention have unexpectedly found that glutaryl histamine is effective in the treatment of respiratory tract diseases such as sinusitis, tonsillitis and acute respiratory distress syndrome (ARDS), by potentiating the effectiveness of antibacterial drugs, shortening the duration and reducing the severity of a disease. Furthermore, it has been found that glutaryl histamine potentiates the effectiveness of antibacterial therapy in the treatment of respiratory tract diseases such as tonsillitis and pneumonia. Such effects of glutaryl histamine were not known and not suggested before.

Thus, the object of the invention is to provide a novel and effective agent for use in the treatment of respiratory tract diseases.

In particular, the object of the invention is to provide an effective agent for use in the treatment of respiratory tract diseases, which agent potentiating the action of antibacterial drugs.

An additional object of the invention is to increase the effectiveness of antibacterial therapy of respiratory tract diseases caused by microorganisms with a reduced sensitivity to antibacterial drugs or by microorganisms resistant to antibacterial therapy.

### Summary of the invention

The above objects of the invention are addressed by the subject matters set forth in the appended claims.

Generally, the invention relates to a medicament for use in the treatment of respiratory tract diseases selected from the group consisting of sinusitis, tonsillitis and acute respiratory distress syndrome (ARDS), the medicament comprising glutaryl histamine or a pharmaceutically acceptable salt thereof in an effective amount. The structural formula of the compound is the following.

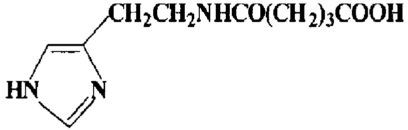

Preferably, the medicament potentiates the effectiveness of antibacterial therapy in the treatment of respiratory tract diseases. Said antibacterial therapy can comprise administration of at least one antibacterial drug selected from the group including cefotaxime, midecamycin, azithromycin, amoxicillin, levofloxacin, oxacillin, vancomycin, and ceftriaxone. In addition, the antibacterial therapy is targeted to microorganisms with a reduced sensitivity to antibacterial drugs or to microorganisms resistant to antibacterial therapy. Said microorganisms can include at least one species selected from *Staphylococcus aureus, Streptococcus pneumonia, Haemophylus influenza* and *Moraxella catarrhalis.*

Further, the invention relates to a pharmaceutical composition for use in the treatment of a respiratory tract disease selected from the group including sinusitis, tonsillitis and acute respiratory distress syndrome (ARDS), wherein the pharmaceutical composition comprises glutaryl histamine or a pharmaceutically acceptable salt thereof in an effective amount, and a pharmaceutically acceptable carrier or diluent. Preferably, the pharmaceutical composition is intended to potentiate the effectiveness of antibacterial therapy in the treatment of respiratory tract diseases. Said antibacterial therapy can comprise administration of at least one antibacterial drug selected from the group including cefotaxime, midecamycin, azithromycin, amoxicillin, levofloxacin, oxacillin, vancomycin, and ceftriaxone. In addition, the antibacterial therapy is targeted to microorganisms with a reduced sensitivity to antibacterial drugs or to microorganisms resistant to antibacterial therapy. Said microorganisms can include at least one species selected from *Staphylococcus aureus, Streptococcus pneumonia, Haemophylus influenza* and *Moraxella catarrhalis.*

The invention further relates to the medicament or pharmaceutical composition for use in potentiating the effectiveness of antibacterial therapy in the treatment of respiratory tract diseases, wherein the respiratory tract disease is preferably selected from the group consisting of tonsillitis and pneumonia.

The composition or medicament for use in the treatment of a respiratory tract disease can be administered in the amount providing the dose of glutaryl histamine or a pharmaceutically acceptable salt thereof of 0.1-100 mg/kg of body weight, preferably 0.5-50 mg/kg of weight body. In a preferred embodiment, the composition or medicament for use according to the invention is administered orally. The composition or medicament for use according to the invention can be administered daily.

The invention also relates to glutaryl histamine or a pharmaceutically acceptable salt thereof for use in the treatment of a respiratory tract disease selected from the group including sinusitis, tonsillitis and acute respiratory distress syndrome (ARDS). Preferably, glutaryl histamine or the pharmaceutically acceptable salt thereof is intended to potentiate the effectiveness of antibacterial therapy in the treatment of respiratory tract diseases. Said antibacterial therapy can comprise administration of at least one antibacterial drug selected from the group including cefotaxime, midecamycin, azithromycin, amoxicillin, levofloxacin, oxacillin, vancomycin, and ceftriaxone. In addition, the antibacterial therapy is targeted to microorganisms with a reduced sensitivity to antibacterial drugs or to microorganisms resistant to antibacterial therapy. Said microorganisms can include at least one species selected from *Staphylococcus aureus, Streptococcus pneumonia, Haemophylus influenza* and *Moraxella catarrhalis.* In a preferred embodiment, a dose of glutaryl histamine or a pharmaceutically acceptable salt thereof is 0.1-100 mg/kg of body weight. A dose of glutaryl histamine or a pharmaceutically acceptable salt thereof can be 0.5-50 mg/kg of weight body.

The invention further relates to glutaryl histamine or a pharmaceutically acceptable salt thereof for use in the treatment of sepsis.

Glutaryl histamine can be also used in the form of pharmaceutically acceptable salts produced by reacting, for example, with sodium hydroxide, potassium hydroxide, potassium carbonate, lithium hydroxide, calcium carbonate by routine methods widely described in literature.

### Brief description of drawings

Fig.1. The degree of interstitial edema (scores) in tracheal administration of LPS over time.
Fig.2. The degree of alveolar infiltration (in % of lung section) in intratracheal administration of LPS over time.
Fig.3. The degree of diapedesis of erythrocytes (in % of lung section) in intratracheal administration of LPS over time.
Fig.4. Lung sections of experimental animals (dexamethasone, 5 mg/kg) 24 hours after administration of LPS. Increase in diapedesis of erythrocytes.
Fig.5. Lung sections of experimental animals (dexamethasone, 5 mg/kg) 72 hours after administration of LPS. Redaction in peribronchial infiltration and interstitial edema.
Fig.6. Lung sections of experimental animals (glutaryl histamine, 27 mg/kg) 24 hours after administration of LPS. Reduction in peribronchial infiltration and interstitial edema.
Fig.7 Lung sections of experimental animals (glutaryl histamine, 27 mg/kg) 72 hours after administration of LPS. Reduction in peri- and intrabronchial infiltration and interstitial edema.
Fig.8. Evaluation of the severity of a clinical symptom (headache) in scores in patients suffering from tonsillitis in various treatment regimens.
Fig.9. Evaluation of the severity of a clinical symptom (fatigue) in scores in patients suffering from tonsillitis in various treatment regimens.
Fig.10. Evaluation of the severity of clinical symptom (sore throat) in scores in patients suffering from tonsillitis in various treatment regimens.
Fig.11. Evaluation of the severity of clinical symptom (purulent deposits) in scores in patients suffering from tonsillitis in various treatment regimens.
Fig.12. Evaluation of the severity of clinical symptom (cough) in scores in patients suffering from tonsillitis in various treatment regimens.
Fig.13. Evaluation of the severity of clinical symptom (rhinitis) in scores in patients suffering from tonsillitis in various treatment regimens.

### EXAMPLES

### Reference Example 1. Effectiveness of glutaryl histamine for the treatment of rhinosinusitis

Activity of glutaryl histamine was studied in an experimental model of acute rhinosinusitis (ARS).

This reference example shows a specific pharmacological activity of glutaryl histamine in an experimental rat model of acute rhinosinusitis induced by intranasal administration of formalin. Administration of 20 µl of 7.5% formalin to the rat nasal passages leads to the development of a clinical picture similar to the symptoms of acute rhinosinusitis in a human.

The tested medicament and reference medicaments were administered for 7 days after administration of 7.5% formalin. Normal saline solution was used as placebo. The animals were subjected to euthanasia on day 8 after induction of experimental acute rhinosinusitis by method of air embolism. During necropsy, nasal passages of all animals were sampled. The obtained material was placed into 10% neutral-buffered formalin for further histological analysis.

The mucous and submucous membranes of both nasal passages (respiratory and olfactory regions) of the experimental animals were subjected to a morphological analysis of the specific activity of glutaryl histamine.

After the clinical phase of the experiment, the material derived from the animals was subjected to the standard treatment to prepare histological paraffin sections with a thickness of 3 to 5 µm. The sections were stained with hematoxylin and eosin for a microscopic study. Comparison and histological analysis of changes were performed versus the group of intact rats.

The main pathological processes inherent in experimental acute rhinosinusitis, such as vascular congestion in mucous membrane, hyperplasia and necrosis of the epithelium, the number of caliciform cells within 1 mm of the mucous membrane in the nasal septum, were evaluated by a semi-quantitative method to evaluate the degree of pathophysiological changes in histological, histochemical and morphological studies.

Body weight was increased in the group treated with the medicament and in the intact and control groups on average by 6.3%, except the animals who received dexamethasone as a therapy, which body weight decreased by 34% due to wasting that was most probably caused by the toxic action of the medicament (see Table 1).

**Table 1**

| Body weight of the experimental animals before the study and after completion of the administration of the medicaments, g, M±m, n=10 | | | | | | | |
|---|---|---|---|---|---|---|---|
| Group | Intact | Control | Diclofenac 11 mg/kg | Dexamethasone 5 mg/kg | Glutaryl histamine 9 mg/kg | Glutaryl histamine 27 mg/kg | Glutaryl histamine 45 mg/kg |
| Initial body weight | 200±5 | 211±5 | 235±6 | 238±5 | 212±3 | 211±4 | 222±4 |
| Body weight on day 8- 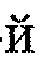 | 215±5 | 221±4 | 236±5 | 157±4* | 227±5 | 227±5 | 245±4 |

| | | | | | | | |
|---|---|---|---|---|---|---|---|
| * - differences are statistically significant compared with the initial body weight (p<0.05) | | | | | | | |

The specific pharmacological activity of medicament glutaryl histamine in various doses compared with diclofenac and dexamethasone was evaluated by histological and histochemical analysis of the effect on production of mucopolysaccharides and caliciform cells.

Acute rhinitis in rats was manifested in the form of mucous and mucopurulent nasal catarrh (Table 2). The main processes characterizing damage in the nasal passages (mucous membrane haemorrhage, hyperproduction of acidic mucous, local necrosis of the epithelium) were apparent in the rats of the control group. The nasal passages of the intact animals did not have macroscopic and microscopic symptoms of damage.

**Table 2**

| Comparative macroscopic characteristic of changes of the mucous membrane in the nasal passages in rats from different groups | | | |
|---|---|---|---|
| Group | Without changes | Mucous catarrh | Mucopurulent catarrh |
| Intact | 10 | 0 | 0 |
| Control | 0 | 2 | 8 |
| Diclofenac 11 mg/kg | 0 | 3 | 7 |
| Dexamethasone 5 mg/kg | 9 | 1 | 0 |
| Glutaryl histamine 9 mg/kg | 3 | 4 | 3 |
| Glutaryl histamine 27 mg/kg | 8 | 1 | 1 |
| Glutaryl histamine 45 mg/kg | 10 | 0 | 0 |

In the group treated with the reference medicament diclofenac, three rats had macroscopic features of mucous catarrh and four animals had more severe disorder, in particular mucopurulent catarrh of the nasal passages, which characterized the clinic picture of acute rhinitis and allowed the conclusion about ineffectiveness of this medicament.

The second reference medicament dexamethasone had more pronounced therapeutic effect since the mucous membrane in the nasal passages did not have macroscopic changes in nine from ten animals.

Evaluation of the effectiveness of glutaryl histamine in various doses has shown that the most pronounced effect of the medicament is observed in doses 27 and 45 mg/kg. In the first case, only two from ten animals had changes in the mucous membrane. The nasal passages of these animals comprised mucus and had rough and flush mucous membrane. The nasal passages of the rats administered glutaryl histamine in a dose of 45 mg/kg did not have symptoms of disorder, in particular, the mucous membrane was pale pink, shiny, and smooth.

Thus, these data demonstrate that dexamethasone in a dose 5 mg/kg and glutaryl histamine in doses of 27 and 45 mg/kg have the maximum effect. However, reference medicament diclofenac at in a dose of 11 mg/kg has an insignificant effect.

The microscopic analysis of exudative disorder developed in rats with acute rhinitis was aimed at measuring the number of caliciform cells comprising acidic mucus (Table 3).

**Table 3**

| The number of caliciform cells within 1 mm of the mucosal membrane of the nasal septum in rats of different groups, M±m, n=10 | | | | | | | |
|---|---|---|---|---|---|---|---|
| Group | Intact | Control | Diclofenac 11 mg/kg | Dexamethasone 5 mg/kg | Glutaryl histamine 9 mg/kg | Glutaryl histamine 27 mg/kg | Glutaryl histamine 45 mg/kg |
| The number of caliciform cells within 1 mm of the mucosal membrane | 14,9±0,5 | 45,6±1,4* | 31,8±1,9* ** | 15,6±0,6** | 29,0±2,7* ** | 19,6±2,4** | 23,9±1,7* ** |

| | | | | | | | |
|---|---|---|---|---|---|---|---|
| Note: * - differences are statistically significant compared with the intact group (p<0.05). ** - differences are statistically significant compared with the intact group (p<0.05). | | | | | | | |

The form and number of caliciform cells depends on the functional condition of the mucous membrane. In catarrh of the nasal mucous membrane, the number of caliciform cells increases, thereby changing their normal ratio to ciliated cells, which destroys the function of the mucociliary transport system providing the movement of the mucous secretory products and different microorganisms and foreign particles, which sediment on its surface, toward the nasopharynx, i.e. the clearance thereof.

Data given in Table 3 demonstrate a pronounced pathological process in the animals of the control group, in particular, the olfactory epithelium in the nasal passages was hyperplastic due to an increased cell ratio, the submucosal glands were enlarged, the number of caliciform cells comprising acidic mucus was significantly increased (p<0.05) three times compared with the group of intact animals (Table 3).

The use of reference medicament diclofenac did not result in a significant effect. The number of caliciform cells significantly increased, almost two times as compared with the group of intact rats, however, valid differences (p<0.05) were also revealed in comparison with the control group, almost one and half times (Table 3). The use of reference medicament dexamethasone in a dose of 5 mg/kg resulted in a pronounced effect, in particular, the number of caliciform cells reaches the number of the same cells in the intact group (Table 3). However, the number of caliciform cells comprising acidic mucus significantly (p<0.05) exceeded their number in the intact group one and half times, but was significantly lower compared with the number of the control group (Table 3).

In the group of animals administered glutaryl histamine in a dose of 27 mg/kg, the number of caliciform cells comprising acidic mucus was insignificantly higher compared with the group of intact rats and was 19.6±2.4, without significant differences (p>0.05), but was significantly lower compared with the control group (p<0.05) (Table 3).

Thus, histological analysis of the nasal meatuses of the rats in experimental model of acute rhinosinusitis has shown a pronounced therapeutic effect of glutaryl histamine in doses of 27 and 45 mg/kg, characterized by regeneration of the epithelium, and reduction in the number of caliciform cells. It has been found that glutaryl histamine in a dose of 45 mg/kg has the effect comparable with that of the reference medicament sand even more beneficial, and this effect has an obvious therapeutic action. At the same time, in spite of an expressed local action, dexamethasone has an apparent toxic action manifested in animal wasting.

### Example 2. Effectiveness of glutaryl histamine for the treatment of tonsillitis

This example is aimed at evaluating the activity of glutaryl histamine on experimental model of tonsillitis. LPS-induced tonsillitis is one of the most adequate models for the study of the pathogenesis, pathomorphological changes and action of pharmacological medicaments on the course of this disease.

Tonsillitis was induced under anesthesia by administration of a solution of *E.coli* lipopolysaccharide (LPS) (Sigma) in normal physiological solution in a dose of 20 µg/kg, by 10 µl to the right upper lymph node (*Inn. Cervicales superficiales*). After administration, the lymph nodes were put under superficial neck muscles and then the skin was stitched. The wound was treated with streptocide. The animal was placed into a post-operation cage.

The tested medicament was administered once daily in a given dose strictly at the stated time for three days before the induction of tonsillitis and 10 days after. Normal saline solution was used as placebo.

Immediately after euthanasia, the animals were subjected to necropsy, the affected and intact lymph nodes were removed and weighed.

The lymph nodes derived from the half of animals from each group were being frozen after removal in a refrigerating chamber at -25°C for 72 hours. Then the lymph nodes were lyophilized.

In a half of animals from each group (7), the affected lymph nodes were subjected to histological analysis. After euthanasia, intact and affected lymph nodes were removed from each animal. After the standard histological treatment with ascending alcohols (70-95%) and impregnation with chloroform, the tissue was embedded with paraffin. Sections with a thickness of 4 to 6 µm were prepared from the paraffin blocks, stained with hematoxylin and eosin to reveal typical pathological processes and study required parameters using light-optical microscope Leica DM LS (magnification 200x and 400x).

Fatal cases were observed in experimental groups treated with the reference medicaments (Table 4). During the experiment, the animals treated with diclofenac had a mortality rate of 29%, whereas the animals treated with dexamethasone had a mortality rate of 64%. Necropsy demonstrated that the death of the animals was caused by intoxication. The animals were too wasted.

**Table 4**

| Mortality in experimental groups | | |
|---|---|---|
| Group | The number of died animals/initial number of animals in a group | mortality rate (%) |
| Intact | 0/14 | 0 |
| Control | 0/14 | 0 |
| Diclofenac | 4/14 | 29 |
| Dexamethasone | 9/14 | 64 |
| Glutaryl histamine 9 mg/kg | 0/14 | 0 |
| Glutaryl histamine 27 mg/kg | 0/14 | 0 |

Weights of intact and affected lymph nodes were compared and the nodes also were lyophilized to evaluate an effect of the medicaments on the swelling degree and adequacy of the model of tonsillitis.

Significantly pronounced swelling of the lymph nodes was observed in the control group - the difference of the weights between the affected and intact lymph nodes was significantly higher, four times more than in the intact group (Table 5). In addition, the animals of the same group had a significant weight loss in the affected lymph nodes after lyophilization, which indicates an apparent preceding exudation and adequately induced local lesion.

**Table 5**

| Weight difference between affected and intact lymph nodes, M±m | |
|---|---|
| Group | Weight difference between affected and intact lymph nodes, mg |
| 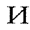 HTaκTHa 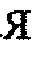 | 3.4±0.3 |
| | n=14 |
| Control | 15.9±0.8* |
| | n=14 |
| Diclofenac | 16.3±1.4* |
| | n=10 |
| Dexamethasone | 15.6±1.8* |
| | n=5 |
| Glutaryl histamine, 9 mg/kg | 13.0±1.3* |
| | n=14 |
| Glutaryl histamine, 27 mg/kg | 8.8±1.1* ** |
| | n=10 |

| | |
|---|---|
| Note: * - differences are statistically significant compared with the intact group (p<0.05). ** - differences are statistically significant compared with the control group (p<0.05). | |

The treatment with the reference medicaments (diclofenac and dexamethasone) did not show any effect.

A significant weight differences between the affected and intact lymph nodes of the animals treated with glutaryl histamine was observed in animals administered glutaryl histamine in a dose of 27 mg/kg (Table 5). At the same time, the differences were significant compared with both the intact group and the control group (p<0.05).

The lymph nodes of the control group had the pattern of acute nonspecific lymphadenitis. Their histological structure was unchanged; in the cortical layer, follicles were enlarged (hyperplasia) and the number of follicles with germinal centers was increased. The area of germinal centers in the lymph nodes was significantly increased, almost two times, compared with the intact group.

**Table 6**

| The area of germinal centers of lymphoid follicles in affected cervical lymph nodes in different groups of rats, M±m | |
|---|---|
| Group | Area of germinal centers of lymphoid follicles in affected lymph nodes, mm² |
| Intact | 0.047±0.002 |
| n=7 | |
| Control | 0.088±0.007* |
| n=7 | |
| Diclofenac | 0.074±0.007* |
| n=5 | |
| Dexamethasone | 0.016±0.004* ** |
| n=3 | |
| Glutaryl histamine, 9 mg/kg | 0.067±0.006* |
| n=7 | |
| Glutaryl histamine, 27 mg/kg | 0.048±0.003** |
| n=7 | |
| Glutaryl histamine 45 mg/kg | 0.066±0.007* |
| n=7 | |

| | |
|---|---|
| * - differences are statistically significant compared with the intact group (p<0.05) ** - differences are statistically significant compared with the control group (p<0.05) | |

The lymph nodes in the animals administered diclofenac, the lymphadenitis pattern was slightly less severe compared with the control group, but without statistically significant differences (Table 6). This medicament did not demonstrate a therapeutic effect.

The study of an effect of dexamethasone on the condition of lymph nodes has shown a distinct statistically significant decrease in both studied parameters. At the same time, the area of the germinal centers of lymph nodes was substantially and significantly less than the area in the intact group.

The lymph nodes of these groups still had the pattern of acute nonspecific lymphadenitis expressed in hyperplasia of cortical lymphoid follicles due to secondary follicles. The primary follicles without light germinal centers were singular. The area of germinal centers increased in affected lymph nodes. The different doses of the medicament have been found to have different effects on the morphological structure of the lymph nodes. In general, the action of glutaryl histamine was expressed in a decrease in the area of the germinal centers of the secondary follicles, which action depended on a dose. Thus, it follows that glutaryl histamine in a dose of 27 mg/kg had the most evident effect. This case had a statistically significant decrease in the area of germinal centers, but without any difference from that in the intact group.

The protein-synthetic function of the liver and a systemic effect of the medicaments on organism were evaluated in total protein (TP) analysis.

**Table 7**

| Evaluation of the blood (TP) of experimental animals, M±m | |
|---|---|
| Group | TP, g/l |
| Intact | 69.0±0.7 |
| | n=11 |
| Control | 59.1±0.5^{*} |
| | n=13 |
| Diclofenac | 60.9±1.2^{*} |
| | n=7 |
| Dexamethasone | 53.0±1.9^{*}_{**} |
| | n=5 |
| Glutaryl histamine, 9 mg/kg | 67.2±1.1_{**} |
| | n=6 |
| Glutaryl histamine 27 mg/kg | 65.0±0.6^{*}_{**} |
| | n=9 |
| Glutaryl histamine 45 mg/kg | 65.1±0.8^{*}_{**} |
| | n=10 |

| | |
|---|---|
| Note: * - differences are statistically significant compared with the intact group (p<0.05) ** - differences are statistically significant compared with the control group (p<0.05) | |

A reduction in the level of total protein was observed in all experimental groups compared with the intact group; the differences were statistically significant (p<0.05). In the group of animals administered reference medicament diclofenac, the TP level was close to the level of the control group and did not have any statistically significant differences (p>0.05). The group of animals administered reference medicament dexamethasone showed a pronounced protein catabolic effect. The TP levels were significantly lower than in the intact and control groups of animals (p<0.05). As for tested medicament glutaryl histamine, the TP levels in all groups administered with different doses were significantly higher than the TP level in the control group. However, in the group "glutaryl histamine, 9 mg/kg", the level did not differ from the level in the intact group and was within the normal range.

Thus, apparent pathological changes characterizing the development of tonsillitis was obtained in the experiment on male Wistar rats by administration of LPS (lipopolysaccharide of *E.coli* outer wall) directly to the tissue of one of the double lymph nodes in a rat. The induced pathology was characterized, first of all, by local changes in the lymph nodes, in particular, by hyperplasia and an increase in the number of follicles with germinal centers. Reference medicament diclofenac in a dose of 11 mg/kg did not have an effect since almost all parameters of this group did not have differences from those in the control group, and the mortality rate in this group was 29%. The effect of dexamethasone in a dose of 5 mg/kg was mainly toxic and immunesuppressive.

The use of tested medicament glutaryl histamine in doses 9 and 27 mg/kg was accompanied by more slightly apparent swelling of the affected lymph node tissue, especially in the second case. In addition, the group of the animals administered glutaryl histamine in a dose of 27 mg/kg demonstrated a decrease in the area of follicular germinal centers, and the values of the total protein level were close to those in the intact group.

### Reference Example 3. Effectiveness of glutaryl histamine for the treatment of bronchiolitis and pneumonia

The therapeutic effectiveness of glutaryl histamine was studied on an experimental model of acute bronchitis. The therapeutic effectiveness of glutaryl histamine was studied on an experimental model of acute bronchitis induced by endotracheal administration of lipopolysaccharide to laboratory Wistar rats through a probe. The probe had an attached syringe filed with a ready solution of LPS (500 µg/animal dissolved in 200 µl of 0.9% NaCl [Nathens A.B. et al., 1998]). After administration of LPS, from 5 to 7 motions by the syringe plunger was made for better distribution of LPS in the pulmonary tract. After this manipulation, the animals were monitored for 2 hours to reveal complications after the administration.

Dexamethasone in a dose of 5 mg/kg was used a reference medicament.

It has been found that LPS caused a strong response of different segments of the lower respiratory tract, the initial stages of which corresponded to acute bronchiolitis (AB) with a rapid progression to acute pneumonia of various severity: from microfocal to macrofocal confluent pneumonia with abscess formations. LPS-induced lung injury caused both systemic and local reactions expressed by interstitialedema. Daily intestinal administration of glutaryl histamine led to improvement of histological symptoms of pneumonia.

Histology and morphometry. Respiratory and air pathways were studied on samples taken from the middle third (hilum of the lung) and apexes of the left lung. Sections of a thickness of 4 to 6 µm were made from the paraffin blocks prepared by the standard method, stained with hematoxylin and eosin for revealing basic pathological processes and studying required parameters by light microscopy. Material for the analysis of the lung histological structure in all animals was taken from the hilum and apex of the lung to study respiratory and air pathways.

The lungs of intact animals do not have macro- and microscopic symptoms of damages, bronchi and bronchioles were lined with simple epithelium. Peribronchial infiltration, which is a variant of the norm, was observed around large bronchi. Exudate and cell detritus specific for destructive processes in the tissue were not found in the lumens of bronchi and bronchioles. Respiratory areas looked aerial, most part of alveolar walls were not thickened. The symptoms of serose or hemorrhagic exudate in the alveolar lumens were not detected.

In the control animals, morphological changes at different time points, starting from the administration of LPS, were characterized by an evident tendency to the dynamic progression of injury and an increase both in the injury severity and in its total area. Thus, the main pathomorphological phenomenon on the first day was peribronchial and bronchial infiltration of middle and small bronchi and bronchioles, which allowed the diagnosis of bronchiolitis in these animals. On the first day these animals also had pronounced interstitial edema with infiltration and thickening interalveolar septa. In some cases there were pronounced alveolar infiltration, vasodilation and vascular congestion, diapedesis of erythrocytes and hemorrhage.

The symptoms of aggravation of the pathological process were observed on the second day after induction of AB. Thus, the degree of the found interstitialinfiltration leading to a decrease in the respiratory surface was larger. These processes support the transition into the stage of lung injury.

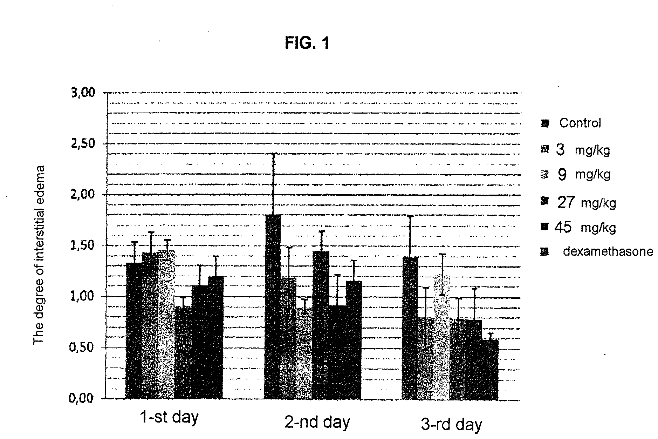

Fig.1 shows the degree of interstitial edema (in scores) in intratracheal administration of LPS over time.

* - differences are statistically significant compared with the control group (p<0.05).

72 hours after administration of LPS, the histological pattern was characterized even severe injury of the respiratory area with the formation of large confluent pneumonia lesions both around bronchi and bronchioles and at a distance from them, covering in some cases up to 50% of the total lung section, thereby abruptly reducing the area of the respiratory surface.

The time history of changes in lungs after administration of LPS are shown in Figs 1, 2 and 3, wherein the changes are expressed by semi-quantitative scores (intensity of interstitial edema expressed by scores) or by a percentage ratio of the injured part of the lung.

Interstitial edema is a thickening of interalveolar septa due to exudation and infiltration by erythrocytes that further may release into the interalveolar space. In an increase of the degree of interstitialedema, the total area of the respiratory surface decreases due to reduction of alveoli, thereby leading to respiratory failure. Interstitial edema was most pronounced in the control on the second day after the administration of LPS, although it was intensive on the other days as well.

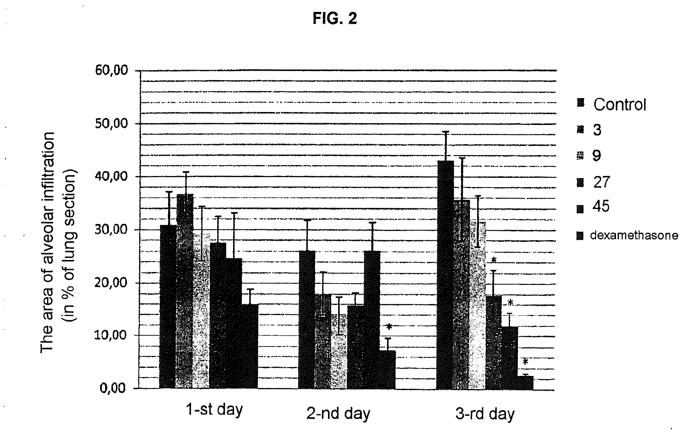

Fig.2 shows the degree of alveolar infiltration expressed in % based on the total area of the lung section, over time.

* - differences are statistically significant compared with the control group (p<0.05).

As can be seen on the figure, the degree of lung injury after the intratracheal administration of LPS was about 30% with an insignificant reduction on the second day, but with a slightly increase on the third day.

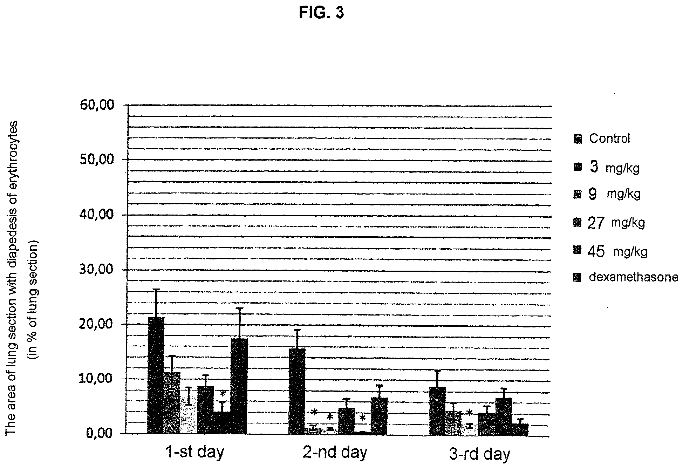

Fig.3 shows the degree of diapedesis of erythrocytes (in % of a lung section) in intratracheal administration of LPS over time.

* - differences are statistically significant compared with the control group (p<0.05).

As was already noted above, the release of erythrocytes into the alveolar lumen was also observed after intratracheal administration of LPS. Fig.3 shows this process over time, where it can be seen that diapedesis gradually decreases and has the minimal value on the third day.

2-3 days after administration of LPS, the group of rats treated with the reference medicament felt themselves worse compared with the control group. It was evident in their inertia, week reaction to an experimenter, hair untidy, and eye crusting. Some of them, like in the control group, were suffered from severe progressing dyspnea with lethal outcome. The cause of death of such animals seemed to be acute hemorrhagic pneumonia complicated by shock with the corresponding morphological changes disclosed above.

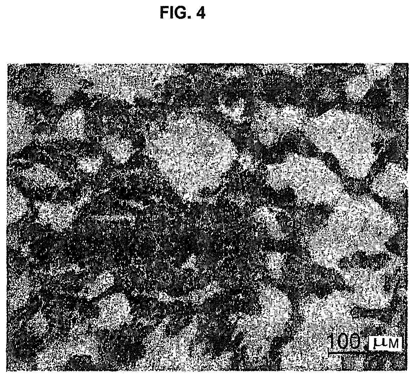

The rest of the rats had lung changes specific for acute lung injury; however, they were much less evident than in the control group. Thus the degree of interstitial edema (Figs 1) was significantly less on the first and second days, and on the third day it was less twice than in the control group. The area of alveolar infiltration (Fig.2) also was initially less, and on the second and third days a difference with the control animals was statistically significant. In addition, on the first day diapedesis of erythrocytes was elevated (Figs 3 and 4), almost the same as in the control group, and decreased on the second day, reaching the minimum value on the third day.

Fig.4 shows lung sections of experimental animals (dexamethasone, 5 mg/kg) 24 hours after administration of LPS. Diapedesis of erythrocytes is elevated.

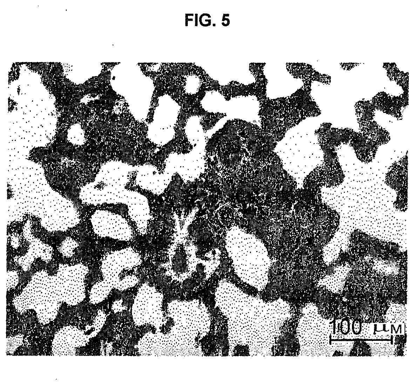

Fig.5 shows lung sections of experimental animals (dexamethasone, 5 mg/kg) 72 hours after administration of LPS. Peribronchial infiltration and interstitial edema are reduced.

Hemorrhagic symptoms in lungs detected at all time points for each tested doses of glutaryl histamine were significantly weaker compared to the control group and the group of dexamethasone.

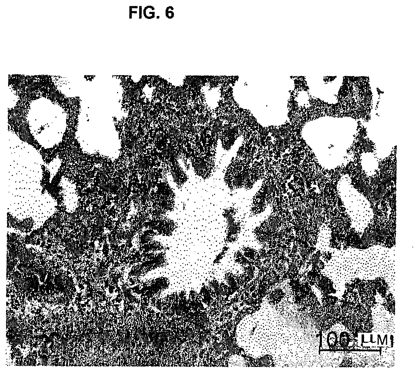

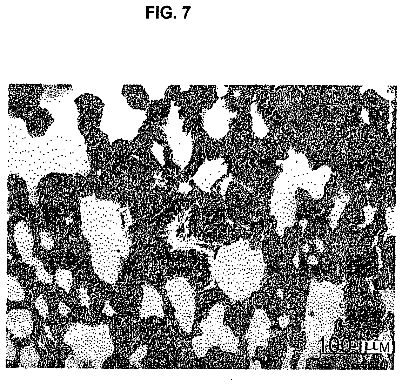

A reduction of interstitial edema was observed in a dose of glutaryl histamine of 27 mg/kg on the first and the third days compared with the control group (Figs 6 and 7). A significant reduction of alveolar infiltration was also observed starting from the second day with maintaining a resultant level up to the third day when the reduction became statistically significant compared with the control group.

Fig.6 shows lung sections of experimental animals (dexamethasone, 27 mg/kg) 24 hours after administration of LPS. Peribronchial infiltration and interstitialedema are reduced.

Fig.7 shows lung sections of experimental animals (dexamethasone, 27 mg/kg) 72 hours after administration of LPS. Peri- and intrabronchial infiltration and interstitialedema are reduced.

Thus, the analysis of various studied parameters has shown a protective action of glutaryl histamine in case of modeling bronchiolitis by endotracheal administration of lipopolysaccharide.

Intratracheal administration of LPS caused massive injuries in different segments of the lower respiratory tracts. The initial stages of the injuries corresponded to acute bronchiolitis with inherent symptoms: the presence of exudate with cell debris inside the respiratory tract and infiltrate of the peribronchiolar and peribronchial areas. Further, the injury rapidly progressed and transformed to acute pneumonia of various severity: from microfocal to macrofocal confluent pneumonia with abscess formations. LPS-induced lung injury caused both systemic and local reactions expressed in interstitial edema.

Compared to dexamethasone, glutaryl histamine had evident advantages such as less pronounced side effects inherent in glucocorticoid hormones. Daily intestinal administration of glutaryl histamine in a dose of 27 mg/kg improved histological symptoms of interstitialedema and hemorrhagic phenomena in the lung tissue.

### Example 4. Effectiveness of glutaryl histamine for the treatment of tonsillitis

The effectiveness of glutaryl histamine for the treatment of lacunar tonsillitis was studied in an open randomized and comparative trial.

The trial included 60 patients which were under medical supervision. When admitted to hospital, patients were randomized into two groups by a random sampling technique. The patients of the first group (30 subjects) received glutaryl histamine in an amount of 90 mg (1 pill) per day for 5 days (450 mg course dose) and standard therapy comprising antibacterial medicaments (penicillin 1 g I.M. 4 times a day or cefasoline 1 g I.M. 3 times a day for 7 days) and nosotropic agents. The patients of the second group (30 subjects) received only standard therapy.

Laboratory analysis included: general blood test, determination of C-reactive protein, and microbiological analysis of tonsil smears. Laboratory data were analyzed on day 6 of the treatment.

Clinical effectiveness of glutaryl histamine was evaluated according to the following criteria: time of temperature normalization; time of disappearance of intoxication symptoms, time-dependent inflammatory changes in the oropharynx, time-dependent changes of laboratory parameters (general blood test and C-reactive protein), complication onset (paratonsillitis, paratonsillar abscess); time-dependent change in symptoms evaluated by a patient and a physician according to a 4-score scale (headache, fatigue, sore throat, purulent deposits in the throat, cough, and rhinitis): 0 - without symptoms, 1 - mild symptom intensity, 2 - moderate symptom intensity, and 4 - severe symptom intensity.

Control time points for the evaluation of the effectiveness of the medicament were treatment days 3, 6 and 8. The treatment withdrawal criteria were normalization of body temperature and the lack of purulent deposits on the tonsils, leukocytosis and neutrophil shift.

Statistical treatment of the obtained results. Values of quantitative variables were expressed as M±SE, wherein M is a mean value, and SE is a standard error of the mean. The validity was confirmed by various modifications of the chi-square test (χ2-test); differences between parameters were considered to be statistically significant (valid) in case of p<0.05. The experimental data were statistically treated using Stat Soft Statistics 6.0 software.

### Results and discussion

The study included 60 18-56 (24.6±1.0 years)-yeas old men the treatment of which began not later than 48 (34.9±1.2 h) hours after symptom onset. All patients had a moderate severity of the disease.

Tonsillar hypertrophy up to 1-2 degrees, swelling of tonsils, and purulent deposits in lacunes were detected. All patients had regional lymphadenitis.

Microflora was represented by non-group A β-hemolytic streptococcus, alpha-hemolytic streptococcus, *Friedlender's bacillus*, *Neisseria* and combinations thereof.

The analysis of demographic data, duration of the disease before the treatment, etiology of respiratory diseases and clinical symptom prevalence rate had demonstrates the absence of statistically significant differences between the compared groups of patients received a complex treatment comprising standard therapy and glutaryl histamine (I group), and patient received only standard therapy (II group).

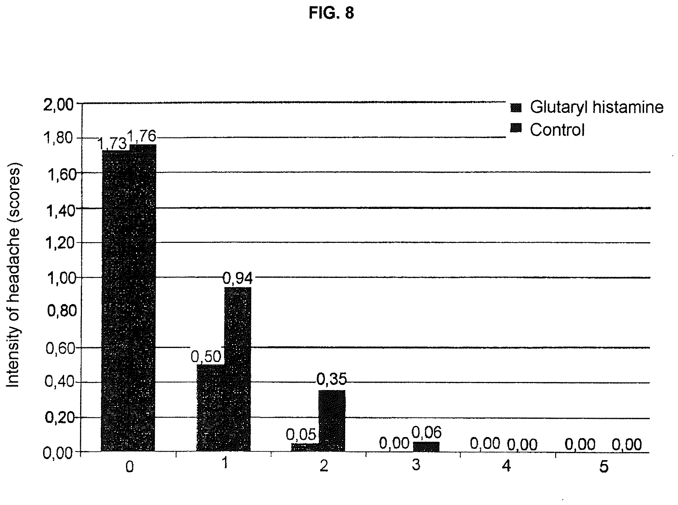

In the first group of patients received glutaryl histamine in the course of the complex treatment, the body temperature normalized within 24 hours in 69.2%, whereas in 33.3% in the second group received standard therapy. In 24-36 hours from the beginning of the treatment, the temperature normalized in 90.0% and 53.3%, respectively (see Table 8). In the control time points (24 and 48 h of the treatment), the mean values of maximum daily body temperature had statistically significant differences between the compared groups (p<0.05; Figs 8-13).

**Table 8**

| Time of the body temperature normalization in tonsillitis-suffered patients in different treatment regimens (n=60) | | | | | |
|---|---|---|---|---|---|
| Time of temperature normalization | Treatment | | | | p* |
| | Standard therapy + glutaryl histamine (I group; n=30) | | Standard therapy (II group; n=30) | | |
| | number | % | number | % | |
| Day 1-1.5 | 27 | 90.0 | 16 | 53.3 | 0.002 |
| Day 2 | 1 | 3,3 | 9 | 30.0 | |
| Day 3 | 2 | 6,7 | 4 | 13.3 | |
| Day 4 | 0 | 0 | 1 | 3.3 | |

| | | | | | |
|---|---|---|---|---|---|
| * - validity of the tendency of later temperature normalization in the group of standard therapy compared with the group treated with glutaryl histamine-containing therapy was evaluated according to Mantel-Haenszel chi-squared test (χ2 test for tendency evaluation). | | | | | |

Intergroup comparison of qualitative values of the body temperature also has shown statistically significant later normalization of temperature in the group of standard therapy (p=0.002).

Analysis of the duration of intoxication symptoms and local changes in the oropharynx (Table 9) demonstrates advantages of the treatment with glutaryl histamine in combination with antibiotics compared with the standard therapy. Shortening in the duration of the main tonsillitis symptoms such as time of clearance of the tonsils from purulent deposits (p=0.007) and sore throat (p=0.003), were statistically significant.

**Table 9**

| Duration of the main clinical symptoms in tonsillitis-suffered patients in different treatment regimens | | | |
|---|---|---|---|
| Symptom | The mean duration, day. | | p* |
| | Standard therapy + glutaryl histamine (I group; n=30) | Standard therapy (II group; n=30) | |
| | M±SE | M±SE | |
| Headache | 1.0±0.1 | 1.1±0.2 | 0.33 |
| Fatigue | 1.2±0.1 | 1.5±0.2 | 0.07 |
| Sore throat | 1.6±0.2 | 2.4±0.2 | 0.003 |
| Purulent deposits in lacunes | 1.8±0.1 | 2.8±0.2 | 0.007 |

| | | | |
|---|---|---|---|
| * - the mean values of symptom duration in two groups of therapy were statistically compared by Student's t-test for quantitative data | | | |

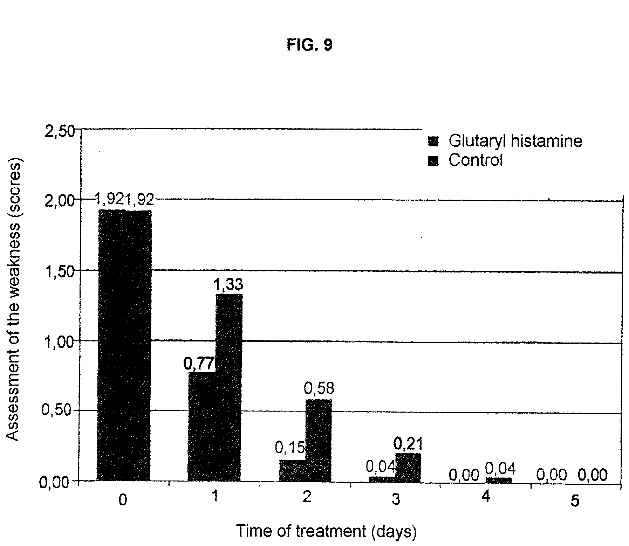

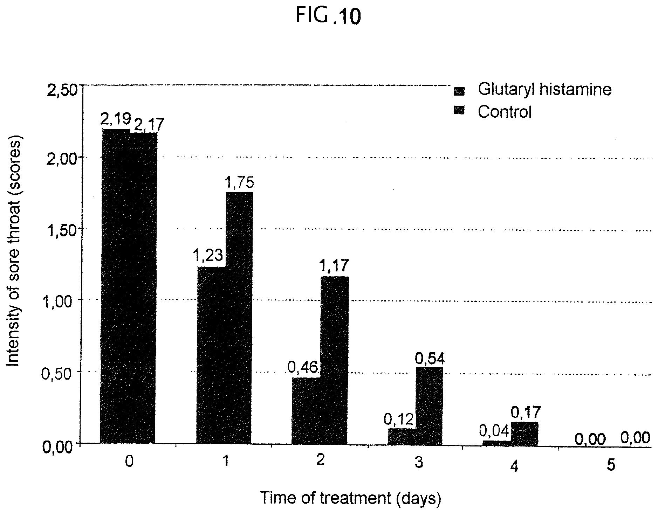

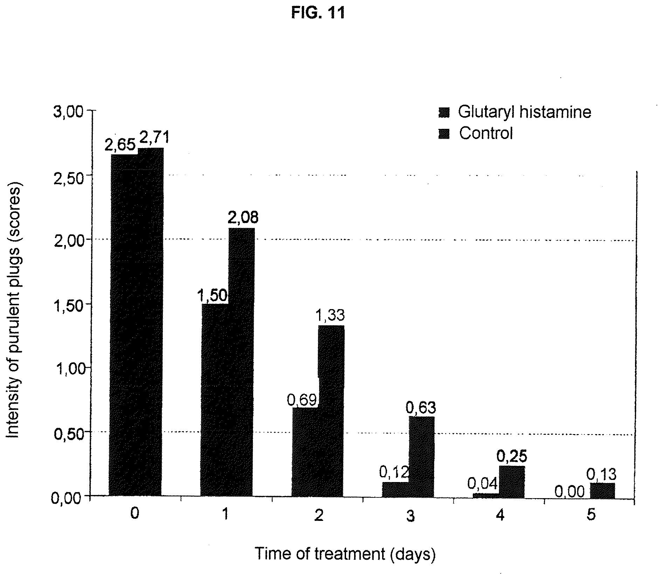

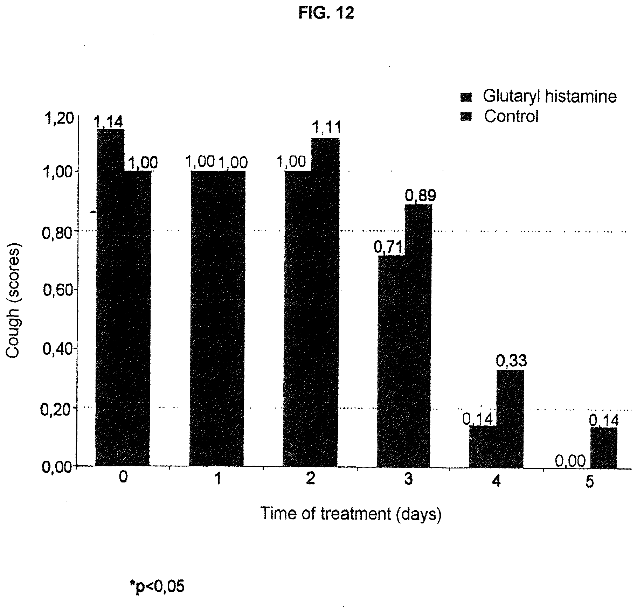

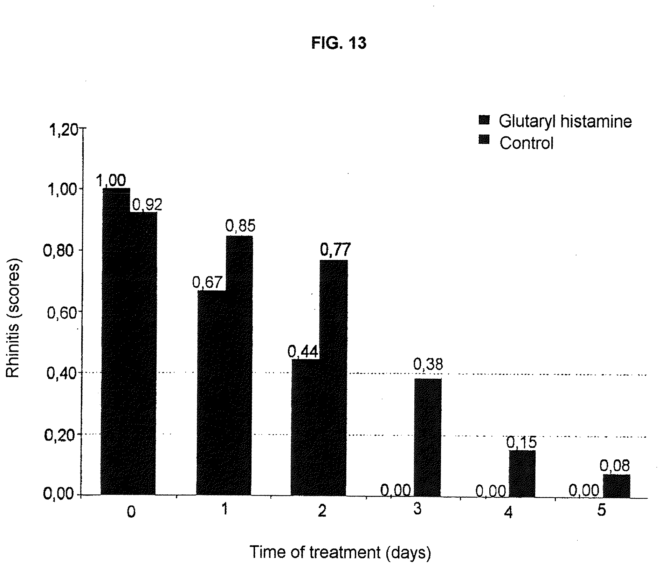

The time period before disappearance of main tonsillitis symptoms (fever, headache, sore throat, purulent deposits in lacunes, and cough) in the group of patients received glutaryl histamine was shorter compared with the group of standard therapy. In accordance with the subjective value of symptoms in scores, patients received glutaryl histamine had valid differences in the headache intensity (Fig.8), fatigue (Fig.9), sore throat (Fig.10), degree of purulent deposits (Fig.11), cough (Fig. 12), and rhinitis (Fig.13).

Before the treatment, blood serum in all patients had an increased level of C-reactive protein, the mean concentration of which decreased more evidently in the patients received glutaryl histamine (48 times on day 6 (control time point after the treatment) compared with the group of standard therapy (12 times) (Table 10).

**Table 10**

| The mean serum concentrations of C-reactive protein in tonsillitis-suffered patients in different treatment regimens (n=60) | | | | | | |
|---|---|---|---|---|---|---|
| Treatment regimen | Concentration of C-reactive protein, µg/ml | | | | | |
| | Before the treatment (1) | Treatment Day 3 (2) | After the treatment (3) | P1-2 | P1-3 | P2-3 |
| Standard therapy + glutaryl histamine (I-group; n=30) | 82.0±12.6 | 31.9±6.9 | 1.7±0.5 | 0.001 | 0.000 | 0.000 |
| Standard therapy (II-group; n=30) | 102.6±12.0 | 31.1±5.4 | 8.6±3.8 | 0.000 | 0.000 | 0.001 |
| p* | 0.4 | 0.8 | 0.077 | | | |

| | | | | | | |
|---|---|---|---|---|---|---|
| * - intergroup differences of the mean values between groups were analyzed by t-test | | | | | | |

Glutaryl histamine was well tolerated by patients. Patients of two groups had no undesirable symptoms and secondary complications (paratonsillitis, abscess). Thetreatment duration of the patients of group I was shorter by one day compared with group II (6.7 and 7.6 days, respectively).

The study of the effectiveness of glutaryl histamine in combined therapy of tonsillitis has shown a high effectiveness and distinct advantages of the combined administration of glutaryl histamine and β-lactam antibiotics.

The use of glutaryl histamine in combined therapy led to shorter duration and severity of fever, reduced intensity of intoxication symptoms and faster disappearance of changes in the tonsillar tissue, all of which were statistically significant compared with standard therapy, thereby shortening the duration of the disease and hospital stay.

Glutaryl histamine has an excellent safety profile - none of the cases had undesirable symptoms.

Thus, the obtained results evidence a high effectiveness and synergistic effects in combined administration of antibacterial drugs and glutaryl histamine.

### Example 5. Effectiveness of glutaryl histamine for the treatment of acute respiratory distress syndrome (ARDS)

Since an intravenous administration of lipopolysaccharide (LPS) to animals is one of the most acceptable models for studying the pathogenesis, pathomorphological changes and consequences of acute respiratory distress syndrome, this model was used in this example. Tested medicament glutaryl histamine was administered for 9 days after the first administration of LPS, i.e. glutaryl histamine was administered for 12 days in total (3 days before the induction of ARDS and 9 days during and after the induction). The exception was dexamethasone since, according to the literature data and the information leaflet, long-time administration of steroidal anti-inflammatory agents can cause severe side effects. For this reason, dexamethasone was administered according to the following schedule: 49, 24 and 1 hour before the induction of ARDS in a dose of 5 mg/kg, for 3 days after the first injection of LPS in the same dose; the medicament was withdrawn by a gradual dose reduction for 3 days. Thus, dexamethasone was administered 9 times.

The mortality rate among the animals administered LPS in an intravenous dose of 7.5 mg/kg was 70% for 8 days. The most of them (60%) died on the first day, in particular, from 4 to 12-16 hours after the injection of LPS. The mortality rate among the animals administered glutaryl histamine was different from the rate in animals treated with dexamethasone; however, most of them also died on the first day, about in the same time period (Table 11).

**Table 11**

| Survival and mortality rates and protective index of different doses of glutaryl histamine and reference medicament dexamethasone | | | | | |
|---|---|---|---|---|---|
| ARDS (mortality rate) | | | | | |
| Group | Total number | Died | Me (%) | S (%) | IP (%) |
| Control | 10 | 7 | 70 | 30 | 0.0 |
| Glutaryl histamine, 0.5 mg/kg | 10 | 3 | 30 | 70 | 57.1 |
| Glutaryl histamine, 3 mg/kg | 10 | 5 | 50 | 50 | 28.6 |
| Glutaryl histamine, 9 mg/kg | 10 | 4 | 40 | 60 | 42.9 |
| Glutaryl histamine, 18 mg/kg | 10 | 5 | 50 | 50 | 28.6 |
| Glutaryl histamine, 27 mg/kg | 10 | 2 | 20 | 80 | 71.4 |
| Glutaryl histamine, 45 mg/kg | 10 | 5 | 50 | 50 | 28.6 |
| Dexamethasone, 5 mg/kg | 10 | 5 | 50 | 50 | 28.6 |

The lowest first-day mortality rate was observed in the groups of the rats administered glutaryl histamine in doses of 0.5 mg/kg and 27 mg/kg. The protective index of glutaryl histamine in doses 0.5 and 27 mg/kg was 57.1% and 71.4%, respectively.

**Table 12**

| Survival and mortality rates and protective index of different doses of glutaryl histamine and the reference medicament dexamethasone | | | | | |
|---|---|---|---|---|---|
| ARDS | | | | | |
| Group | Total number | Died | M (%) | S(%) | IP (%) |
| Control | 24 | 8 | 33.3 | 66.7 | 0.0 |
| Glutaryl histamine, 0.5 mg/kg | 24 | 3 | 12.5 | 87.5 | 62.5 |
| Glutaryl histamine, 27 mg/kg | 24 | 2 | 8.3 | 91.7 | 75.0 |
| Dexamethasone, 5 mg/kg | 24 | 3 | 12.5 | 87.5 | 62.5 |

At this step, animals were administered LPS at more lower dose, but for longer time period. They were received 2 mg/kg on days 1 and 2, and 3 and 4 mg/kg on days 3 and 4, respectively. The maximum mortality rate, similarly to the single-dose administration of LD₇₀, was on the first day. On eighth day, mortality reached 33%. Survival and mortality rates and protective index are given in Table 12.

Thus, the mortality rate among the animals administered glutaryl histamine in doses 0.5 and 27 mg/kg was 12.5 and 8.3%, and protective index was 62.5 and 75%, respectively. The mortality rate among the animals administered dexamethasone was 0% for the first three days, but, starting from the fourth day, 8% of animals died, and additional 4% of animals died on the fourth day. Thus, the mortality rate among the animals administered dexamethasone was 12.5% and protective index was 62.5%.

The injury pattern of lungs was typical for acute respiratory distress syndrome. The maximum intensity was observed four hours after administration of LPS. However, interstitial swelling accompanied by thickening interalveolar septa, alveolar edema with exudate effusion into alveoli, formation of "hyaline membranes" and the presence of insignificant alveolar infiltration were already observed after two hours. Massive diapedesis of erythrocyteswas also detected, and in some cases there were hemorrhage, hyperemia and congestion in small and middle vessels, perivascular edema and perivascular infiltration. Similar symptoms also were detected in the control animals, however, they were pronounced less than 4 hours after. The maximum microscopic ARDS-associated changes in the lungs were in died animals and animals that were dying. These rats had disorders also in other organs: liver, kidneys, and intestinal tract, manifested in a greater or lesser degree, which supported the development of multiple organ failure syndrome.

The analysis of the results has shown that the mortality rate is minimal in a dose of glutaryl histamine of 0.5 mg/kg (30% for 8 days) and 27 mg/kg (20%), protective index is 57.1% and 71.4%, respectively. The mortality rate among the animals administered dexamethasone in a dose of 5 mg/kg stands out. The maximum mortality rate among the control animals and the animals treated with the medicaments was on the first day, namely, first 16 hours after intravenous administration of LPS. Among the rates received dexamethasone, fatal cases were not registered for the first 3 days. However, after the drug withdrawal (administration of all medicaments was terminated on the third day after the injection of LPS), intensive, almost daily death of animals was registered from the fourth day. Therefore, the protective index was 28.6%. The same picture was at the second stage of the experiment when the death of animals treated with dexamethasone was suspended and observed starting from the fourth day. This seems to be associated with side effects of dexamethasone, in particular, with accession of a secondary infection. The accession of a secondary infection was supported, in particular, in autopsy of these animals, in particular, by the formation of multiple abscesses.

The second step of the experiment comprises the evaluation of both the mortality rate of the animals with calculation of the protective index and narrower indexes used for determination of a specific protection provided by glutaryl histamine in ARDS. As was disclosed above, a lower dose of LPS (LD₃₀) was used at the second stage. In addition, LPS was administered every day for 4 days. This method of administration was aimed at achieving the maximum disorder exactly in the lung for subsequent evaluation of possible protective action of glutaryl histamine.

The analysis of the mortality rate among the animals treated with multiple doses of LPS has shown that the protective index of glutaryl histamine in a dose of 27 mg/kg was slightly higher than in a dose of 0.5 mg/kg and was 75% and 62.5%, respectively. This substantially agreed with the data obtained at the first stage of the experiment and evidenced high activity of glutaryl histamine in this pathology. The protective index of dexamethasone also was 62.5%.

Since the mortality rate among the animals treated with glutaryl histamine was significantly lower than in the control animals, it allows the assumption that multiple organ failure was also manifested in a less degree. Thus, in view of the experimental data obtained in the used rat model of acute respiratory distress syndrome caused by a single-dose and multiple-dose intravenous administration of lipopolysaccharide, it has been shown that glutaryl histamine possesses a dose-dependent specific therapeutic activity for six tested doses (0.5, 3, 9, 18, 27 and 45 mg/kg) in intravenous multiple-dose administration of LPS in LD₃₀ dose, respectively. The effectiveness of this drug in combination with less prominent side effects in comparison with dexamethasone allows the conclusion that the administration of glutaryl histamine is preferred than the administration of steroidal anti-inflammatory agents.

### Example 6. Effectiveness of glutaryl histamine in combination with levofloxacin in staphylococcal sepsis and E.coli sepsis in mice

This example is aimed at studying activity of glutaryl histamine in combination with levofloxacin in staphylococcal sepsis and *E.coli* sepsis in mice.

The tested medicament was glutaryl histamine in doses of 15, 30 and 45 mg/kg. Solutions of the tested medicament for oral administration were prepared *ex tempore.* Levofloxacin (in oral administration) was used as a therapeutic preparation since this substance was the most experimentally studied antibiotic approved in clinical practice many years ago.

*Staphylococcus aureus* (mouse-adaptedstrain 10) was used as an infectious agent. Mice were infected intravenously.

Firstly, there was determined a lethal dose (LD₁₀₀) of staphylococcus to be intravenously administered to this line of mice having a certain weight. Fatal cases among mice were registered every day for ten days. The lethal dose was 3×10⁸ CFU/mouse. After that, there was determined ED₅₀ (50% effective dose) of levofloxacin for staphylococcus taken in a lethal dose. For this purpose, mice were housed into cages (10 animals per cage) and infected with a lethal dose of staphylococcus. One hour after each mouse was administered orally levofloxacin in doses of 1, 2, 4, 6, 8, 10, and 12 mg/kg. A group of mice infected with a lethal dose of staphylococcus without the treatment was considered as a control group. Fatal cases among mice were registered every day for ten days. ED₅₀ for levofloxacin was 2.65 mg/kg (2.5 mg/kg in experiment).

The next step of the experiment was to study an effect of combined administration of glutaryl histamine and levofloxacin on a model of staphylococcal sepsis in mice.

In the experiment, mice were housed in following groups (10 animals per cage):
1. Control, lethal dose of staphylococcus
2. staphylococcus + levofloxacin, 2.5 mg/kg
3. staphylococcus + glutaryl histamine, 15 mg/kg
4. staphylococcus + glutaryl histamine, 30 mg/kg
5. staphylococcus + glutaryl histamine, 45 mg/kg
6. staphylococcus + glutaryl histamine, 15 mg/kg + levofloxacin, 2.5 mg/kg
7. staphylococcus + glutaryl histamine, 30 mg/kg + levofloxacin, 2.5 mg/kg
8. staphylococcus + glutaryl histamine, 45 mg/kg + levofloxacin, 2.5 mg/kg

Glutaryl histamine was administered orally in the corresponding doses to the mice of groups 3, 4, 5, 6, 7, and 8 for 5 days. Five day after, all groups of mice were intravenously administered with 0.2 ml of *Staphylococcus aureus* in the lethal dose. One hour after infection, mice of groups 2, 6, 7 and 8 were orally administered 0.2 ml of levofloxacin in a dose of 2.5 mg/kg. After infection and administration of levofloxacin, glutaryl histamine was administered to mice of groups 3-8 for additional 5 days. During the experiment, the animals were observed every day, and fatal cases were registered (see Table 13).

*Escherichia coli* (mouse-adaptedstrain 4300, Institute collection) was used as an infectious agent. Mice were infected intravenously.

Firstly, there were determined a lethal dose (LD₁₀₀) of *Escherichia coli* to be intravenously administered to this line of mice with a certain weight. Fatal cases among mice were registered every day for ten days. The lethal dose was 4×10⁸ CFU/mouse. After that, there was determined ED₅₀ (50% effective dose) of levofloxacin for *Escherichia coli* taken in a lethal dose. For this purpose, mice were housed into cages (10 animals per cage) and infected with a lethal dose of *Escherichia coli.* One hour after each mouse was administered orally levofloxacin in doses of 1, 2, 4, 6, 8, 10, and 12 mg/kg. A group of mice infected with a lethal dose of *Escherichia coli* without the treatment was considered as a control group. Fatal cases among mice were registered every day for ten days. ED₅₀ levofloxacin was 2.97 mg/kg (3 mg/kg in experiment).

The next step of the experiment was to study an effect of combined administration of glutaryl histamine and levofloxacin on a model of *E.coli* sepsis in mice.

In the experiment, mice were housed in following groups (10 animals per cage):
9. Control, lethal dose of Escherichia coli
10. Escherichia coli + levofloxacin, 3 mg/kg
11. Escherichia coli + glutaryl histamine, 15 mg/kg
12. Escherichia coli + glutaryl histamine, 30 mg/kg
13. Escherichia coli + glutaryl histamine, 45 mg/kg
14. Escherichia coli + glutaryl histamine, 15 mg/kg + levofloxacin, 3 mg/kg
15. Escherichia coli + glutaryl histamine, 30 mg/kg + levofloxacin, 3 mg/kg
16. Escherichia coli + glutaryl histamine, 45 mg/kg + levofloxacin, 3 mg/kg

Glutaryl histamine was administered orally in the corresponding doses to mice of groups 3, 4, 5, 6, 7, and 8 for 5 days. Five day after, all groups of mice were intravenously administered 0.2 ml of *Escherichia coli* in the lethal dose. One hour after infection, mice of groups 2, 6, 7 and 8 were orally administered 0.2 ml of levofloxacin in a dose of 3 mg/kg. After infection and administration of levofloxacin, glutaryl histamine was administered to mice of groups 3-8 for additional 5 days. During the experiment, the animals were observed every day, and fatal cases were registered (see Table 14).

**Table 13**

| Evaluation of activity of glutaryl histamine in combination with levofloxacin on the model of staphylococcal sepsis in mice | | | | | | | | | | | | | |
|---|---|---|---|---|---|---|---|---|---|---|---|---|---|
| Tested medicament | Dose, mg/kg | Days of experiment (numerator - died animals, denominator - survived animals) | | | | | | | | | | Mortality rate (%) | Survival rate (%) |
| | | 1 | 2 | 3 | 4 | 5 | 6 | 7 | 8 | 9 | 10 | | |
| St.a. + levofloxacin | 2,65 | 0/10 | 0/10 | 1/9 | 2/8 | 4/6 | 4/6 | 4/6 | 5/5 | 5/5 | 5/5 | 50 | 50 |
| St.a. + glutaryl histamine | 15 | 0/10 | 0/10 | 0/10 | 1/9 | 2/8 | 3/7 | 4/6 | 6/4 | 7/3 | 8/2 | 80 | 20 |
| | 30 | 0/10 | 0/10 | 0/10 | 1/9 | 3/7 | 4/6 | 6/4 | 7/3 | 7/3 | 7/3 | 70 | 30 |
| | 45 | 0/10 | 0/10 | 1/9 | 2/8 | 4/6 | 5/5 | 7/3 | 8/2 | 8/2 | 8/2 | 80 | 20 |
| St.a. + levofloxacin + glutaryl histamine | 2.65+15 | 0/10 | 0/10 | 0/10 | 0/10 | 0/10 | 1/9 | 2/8 | 2/8 | 2/8 | 2/8 | 10 | 80 |
| | 2.65+30 | 0/10 | 0/10 | 0/10 | 0/10 | 0/10 | 0/10 | 0/10 | 0/10 | 0/10 | 0/10 | 0 | 100 |
| | 2.65+45 | 0/10 | 0/10 | 0/10 | 0/10 | 1/9 | 1/9 | 1/9 | 1/9 | 1/9 | 1/9 | 0 | 90 |
| Control, St.a. | Lethal dose | 0/10 | 0/10 | 2/8 | 3/7 | 5/5 | 7/3 | 8/2 | 10/0 | 10/0 | 10/0 | 100 | 0 |

Analysis of data in this table demonstrates that the first fatal case was registered on the third day in the control group, in the group of St.a. + glutaryl histamine (45 mg/kg) and in the group of St.a. + levofloxacin. On the fourth day, fatal cases still occurred in the control group and in the groups of St.a. + glutaryl histamine and St.a. + levofloxacin in all doses. The most fatal cases were observed in the control group and tested groups on the 5^{th}, 6^{th}, and 7^{th} days of the experiment. In the group of St.a. + glutaryl histamine + levofloxacin, one mouse died on the fifth day (in dose of 45 mg/kg); in a dose of 30 mg/kg there were no fatal cases during the whole experiment. The last mouse in the control group died on the eighth day of the experiment. On the tenth day, the survival rate was the following:
group of St.a. + levofloxacin - 50%;
groups of St.a. + glutaryl histamine:
in a dose of 15 mg/kg - 20%,
in a dose of 30 mg/kg - 30%, and
in a dose of 45 mg/kg - 20%;

group of St.a. + glutaryl histamine + levofloxacin:
in a dose of 15 mg/kg - 80%,
in a dose of 30 mg/kg - 100%, and
in a dose of 45 mg/kg - 90%.

**Table 14**

| Evaluation of activity of glutaryl histamine in combination with levofloxacin on the model of *E.coli* sepsis in mice | | | | | | | | | | | | | |
|---|---|---|---|---|---|---|---|---|---|---|---|---|---|
| Tested medicament | Dose, mg/kg | Days of experiment (numerator - died animals, denominator - survived animals) | | | | | | | | | | Mortality rate (%) | Survival rate (%) |
| | | 1 | 2 | 3 | 4 | 5 | 6 | 7 | 8 | 9 | 10 | | |
| E.coli + levofloxacin | 20 | 0/10 | 0/10 | 0/10 | 1/9 | 2/8 | 3/7 | 4/6 | 5/5 | 5/5 | 5/5 | 50 | 50 |
| E.coli + glutaryl histamine | 15 | 0/10 | 0/10 | 0/10 | 1/9 | 2/8 | 5/5 | 6/4 | 8/2 | 8/2 | 8/2 | 80 | 20 |
| | 30 | 0/10 | 0/10 | 0/10 | 1/9 | 3/7 | 6/4 | 7/3 | 7/3 | 8/2 | 8/2 | 80 | 20 |
| | 45 | 0/10 | 1/9 | 1/9 | 3/7 | 5/5 | 7/3 | 8/2 | 9/1 | 9/1 | 9/1 | 90 | 10 |
| E.coli + levofloxacin + glutaryl histamine | 20+15 | 0/10 | 0/10 | 0/10 | 0/10 | 0/10 | 0/10 | 1/9 | 1/9 | 1/9 | 1/9 | 10 | 90 |
| | 20+30 | 0/10 | 0/10 | 0/10 | 0/10 | 0/10 | 1/9 | 1/9 | 1/9 | 1/9 | 1/9 | 10 | 90 |
| | 20+45 | 0/10 | 0/10 | 0/10 | 0/10 | 0/10 | 1/9 | 1/9 | 2/8 | 2/8 | 2/8 | 20 | 80 |
| Control, E.coli | Lethal dose | 0/10 | 0/10 | 1/9 | 3/7 | 4/6 | 6/4 | 8/2 | 10/0 | 10/0 | 10/0 | 100 | 0 |

Analysis of data in this table demonstrates that the first fatal case was registered on the second day in the group of E.coli + glutaryl histamine (45 mg/kg). On the third day, fatal cases occurred in the control group and in the groups of *E.coli* + glutaryl histamine (45 mg/kg). On the fourth day, fatal cases still occurred in the control group and in the groups of *E.coli* + glutaryl histamine in all doses. The most fatal cases were observed in the control group and tested groups on the 5^{th}, 6^{th}, 7^{th}, and 8^{th} days of the experiment. In group of E.coli + glutaryl histamine + levofloxacin, one mouse died on the sixth day (in doses 30 and 45 mg/kg). The last mouse in the control group died on the seventh day of the experiment. On the tenth day, the survival rate was the following:
group of E.coli + levofloxacin - 50%;
groups of E.coli + glutaryl histamine:
   in a dose of 15 mg/kg - 20%,
   in a dose of 30 mg/kg - 20%, and
   in a dose of 45 mg/kg - 10%;
groups of E.coli + glutaryl histamine + levofloxacin:
   in a dose of 15 mg/kg - 90% and
   in doses of 30 and 45 mg/kg - 80%.

Glutaryl histamine in a dose ranging from 15 to 45 mg/kg in a combined therapy increases the efficiency of levofloxacin in staphylococcal sepsis from 50% to 80-100% according to the survival rate, and in *E.coli* sepsis from 50 to 80-90% according to the survival rate, thereby confirming its pronounced potentiating properties.

Given the effect obtained in the administration of various doses of glutaryl histamine in a combined therapy, it can be concluded that a dose of 30 mg/kg seems to be optimal. The maximum effect observed in the combination with levofloxacin was a survival rate of 100% in staphylococcal sepsis and 90% in *E.coli* sepsis.

The obtained results of the use glutaryl histamine in combination with levofloxacin in sensitive mouse lines demonstrate its potentiating action in the lines with hyposensitivity to antibiotics and in the resistant lines (MRSA type).

### Example 7 Effectiveness of a combined administration of glutaryl histamine and ampicillin on a model of staphylococcal sepsis in mice

Before the study, laboratory animals were confined in groups in cages for 14 days for adaptation. During this period, clinical condition of the animals was monitored by visual examination. Animals with abnormalities revealed during the examination were removed from the experimental groups.

Before the study, the animals complying with the inclusion criteria were divided into groups.

Animals without evident abnormalities were randomly divided into groups so that their individual weights did not deviate from the average value more than 10%.

The tested medicament was glutaryl histamine in doses of 15, 30 and 45 mg/kg. Solutions of the tested medicament for oral administration were prepared *ex tempore.* Ampicillin (in intravenous administration) was used as a therapeutic preparation since this substance was the most experimentally studied antibiotic approved in clinical practice many years ago.

*Staphylococcus aureus* (mouse-adapted strain 10) was used as an infectious agent. Mice were infected intravenously.

Firstly, there was determined a lethal dose (LD₁₀₀) of staphylococcus when intravenously administered to this line of mice with a certain weight. Fatal cases among mice were registered every day for ten days. The lethal dose was 3×10⁸CFU/mouse. After that, there was determined ED₅₀ (50% effective dose) of ampicillin for staphylococcus taken in a lethal dose. For this purpose, mice were housed into cages (10 animals per cage) and infected with a lethal dose of staphylococcus. One hour later, each mouse was intravenously administered ampicillin in doses of 10, 20, 30, 40, and 50 mg/kg. A group of mice infected with a lethal dose of staphylococcus without the treatment was considered as a control group. Fatal cases among mice were registered every day for ten days. ED₅₀ ampicillin was 17.3 mg/kg (20 mg/kg in experiment).

The animals were observed for 10 days, and mortality rate was registered every day:

The next step of the experiment was to study an effect of combined administration of glutaryl histamine with ampicillin on a model of staphylococcal sepsis in mice.

In the experiment, mice were housed in following groups (10 animals per cage):
Control, lethal dose of staphylococcus
staphylococcus + ampicillin, 20 mg/kg
staphylococcus + glutaryl histamine, 15 mg/kg
staphylococcus + glutaryl histamine, 30 mg/kg
staphylococcus + glutaryl histamine, 45 mg/kg
staphylococcus + glutaryl histamine, 15 mg/kg + ampicillin, 20 mg/kg
staphylococcus + glutaryl histamine, 30 mg/kg + ampicillin, 20 mg/kg
staphylococcus + glutaryl histamine, 45 mg/kg + ampicillin, 20 mg/kg

Glutaryl histamine was administered orally in the corresponding doses to mice of groups 3, 4, 5, 6, 7, and 8 for 5 days. Five day after, all groups of mice were intravenously administered 0.2 ml of *Staphylococcus aureus* in the lethal dose. One hour after infection, mice of groups 2, 6, 7 and 8 were orally administered 0.2 ml of ampicillin in a dose of 20 mg/kg. After infection and administration of ampicillin, mice of groups 3-8 were administered glutaryl histamine for additional 5 days. During the experiment, the animals were observed every day, and fatal cases were registered.

**Table 15**

| Evaluation of activity of glutaryl histamine in combination with ampicillin on the model of staphylococcal sepsis in mice | | | | | | | | | | | | | |
|---|---|---|---|---|---|---|---|---|---|---|---|---|---|
| Tested medicament | Dose, mg/kg | Days of experiment (numerator - died animals, denominator - survived animals) | | | | | | | | | | Mortality rate (%) | Survival rate (%) |
| | | 1 | 2 | 3 | 4 | 5 | 6 | 7 | 8 | 9 | 10 | | |
| St.a. + ampicillin | 20 | 0/10 | 0/10 | 0/10 | 1/9 | 2/8 | 3/7 | 4/6 | 4/6 | 5/5 | 5/5 | 50 | 50 |
| St.a. + glutaryl histamine | 15 | 0/10 | 0/10 | 0/10 | 1/9 | 2/8 | 4/6 | 5/5 | 6/4 | 6/4 | 7/3 | 70 | 30 |
| | 30 | 0/10 | 0/10 | 0/10 | 1/9 | 2/8 | 3/7 | 4/6 | 6/4 | 7/3 | 7/3 | 70 | 30 |
| | 45 | 0/10 | 0/10 | 1/9 | 1/9 | 3/7 | 4/6 | 5/5 | 6/4 | 7/3 | 8/2 | 80 | 20 |
| St.a. + ampicillin + glutaryl histamine | 20+15 | 0/10 | 0/10 | 0/10 | 0/10 | 0/10 | 1/9 | 1/9 | 1/9 | 1/9 | 1/9 | 10 | 90 |
| | 20+30 | 0/10 | 0/10 | 0/10 | 0/10 | 0/10 | 0/10 | 0/10 | 0/10 | 0/10 | 0/10 | 0 | 100 |
| | 20+45 | 0/10 | 0/10 | 0/10 | 0/10 | 0/10 | 0/10 | 0/10 | 0/10 | 0/10 | 0/10 | 0 | 100 |
| Control, St.a. | Lethal dose | 0/10 | 0/10 | 1/9 | 3/7 | 4/6 | 6/4 | 8/2 | 10/0 | 10/0 | 10/0 | 100 | 0 |

It should be noted that in the experiment, ampicillin was used in a 50% effective dose (ED₅₀) since a positive or negative action of glutaryl histamine can be registered only in such a dose.

Analysis of data in this table demonstrates that the first fatal case was registered on the third day in the control group and in the group of St.a. + glutaryl histamine (45 mg/kg). The fatal cases were also observed on day 4 in the control group and were registered in the group of St.a. + ampicillin, and for all doses in the group of St.+ glutaryl histamine. Most of fatal cases were registered in the control group and tested groups on the 5^{th}, 6^{th}, and 7^{th} days of the experiment. In the group of St.a. + glutaryl histamine + ampicillin, one mouse died on the sixth day (dose of 15 mg/kg); in doses of 30 and 45 mg/kg there were no fatal cases during the whole experiment. The last mouse in the control group died on the seventh day of the experiment. On the tenth day, the survival rate was the following:
group of St.a. + ampicillin - 50%;
groups of St.a. + glutaryl histamine:
   in a dose of 15 mg/kg - 30%,
   in a dose of 30 mg/kg - 30%, and
   in a dose of 45 mg/kg - 20%;
groups of St.a. + glutaryl histamine + ampicillin:
   in a dose of 15 mg/kg - 90% and
   in doses of 30 and 45 mg/kg - 100%.
   Analysis of the obtained results allows the following conclusion.

Combination of ampicillin (in a dose of ED₅₀) with glutaryl histamine in doses 15, 30 and 45 mg/kg resulted in a survival rate of 90 to 100%. This evidences that glutaryl histamine has a potentiating activity to ampicillin.

Thus, given the above experimental data, glutaryl histamine can be used for the treatment of respiratory tract diseases, in particular to enhance the efficiency of antibacterial therapy for the treatment of respiratory tract disease. The diseases can be rhinosinusitis, sinusitis, tonsillitis, bronchiolitis, pneumonia, acute respiratory distress syndrome (ARDS).

### Example 8. Effectiveness of glutaryl histamine in combination with ampicillin in MRSA (methicillin resistant strain)-induced staphylococcal sepsis in rats

This experiment was aimed at evaluating activity of glutaryl histamine in combination with ampicillin on a model of MRSA-induced staphylococcal sepsis.

Levofloxacin was used in a 50% effective dose (ED₅₀) as a positive control.

Before the study, laboratory animals were confined in groups in cages for 14 days for adaptation. Clinical condition of the animals was monitored by visual examination during this period. Animals with abnormalities revealed during the examination were not included into the experimental groups.

Before the study, the animals complying with the inclusion criteria were divided into groups.

Animals without evident abnormalities were randomly divided into groups so that their individual weights did not deviate from the average value more than 10%.

Tested medicament was glutaryl histamine in doses of 15, 30 and 45 mg/kg. Solutions of the tested medicament for oral administration were prepared *ex tempore* in stream water. Ampicillin (in intravenous administration) was used as a therapeutic preparation since it is the most experimentally studied antibiotic approved in clinical practice many years ago. Levofloxacin was used as a positive control (in oral administration).

*Staphylococcus aureus* was used as an infectious agent (mouse-adapted MRSA strain 5, collection from GU NIINA im.G.F.Gauze RAMN).

Firstly, there was determined a lethal dose (LD₁₀₀) of staphylococcus when intravenously administered to this line of mice with a certain weight. Fatal cases among mice were registered every day for ten days. The lethal dose was 8×10⁸ CFU/mouse. After that, there was determined the dose of ampicillin providing a survival rate of 20% of mice infected with staphylococcus MRSA taken in a lethal dose. For this purpose, mice were housed into cages by 10 animals and infected with a lethal dose of staphylococcus. One hour later, each mouse was intravenously administered ampicillin in doses of 30, 60, 90, 120, 150, and 180 mg/kg. A group of mice infected with a lethal dose of staphylococcus without the treatment was considered as a control group. Fatal cases among mice were registered every day for ten days. The dose of ampicillin providing a survival rate of 20% of mice infected with staphylococcus MRSA was 120 mg/ml. Levofloxacin (to which the strain MSRA is sensitive) in a dose of ED₅₀ (4mg/kg) was used as a positive control.

The next step of the experiment was to study an effect of combined administration of glutaryl histamine with ampicilline on a model of MRSA-induced staphylococcal sepsis in mice.

In the experiment, the mice were housed in following groups of 10 animals per cage:
1. Control, lethal dose of staphylococcus MSRA (8×10⁸, i.v., one time)
2. Staphylococcus (8×10⁸, i.v., one time) + ampicillin (120 mg/ml, i.v., one time)
3. Staphylococcus (8×10⁸, i.v., one time) + levofloxacin (4 mg/ml, *per os,* one time)
4. Staphylococcus(8×10⁸, i.v., one time) + glutaryl histamine (15 mg/ml, *per os,* 10 days) + ampicillin (120 mg/ml, i.v., one time)
5. Staphylococcus (8×10⁸, i.v., one time) + glutaryl histamine (30 mg/ml, *per os,* 10 days) + ampicillin (120 mg/ml, i.v., one time)
6. Staphylococcus (8×10⁸, i.v., one time)+ glutaryl histamine (45 mg/ml, *per os,* 10 days) + ampicillin (120 mg/ml, i.v., one time).

### Determination of LP₁₀₀ of Staphylococcus aureus

As follows from the data given in Table 16, the first fatal case in the tested groups was recorded on the third day in a dose 7×10⁸ (1 mouse); and in doses 8 and 9×10⁸ (two mice in each case). The last fatal cases were recorded on the fifth day in a dose of 6×10⁸; on the seventh day in a dose of 9×10⁸; and on the eight day in doses of 7×10⁸ and 8×10⁸. On day 10, the mortality rate was the following: 50% in a dose of 6×10⁸, 80% in a dose of 7×10⁸, and 100% in doses of 8 and 9×10⁸. The control (intact) group had no fatal cases.

**Table 16**

| Determination of LD₁₀₀St.a (MRSA) in mice SHK | | | | | | | | | | | |
|---|---|---|---|---|---|---|---|---|---|---|---|
| Doses St.a. MRSA (CFU/ mouse) | Days of experiment (died/survived animals) | | | | | | | | | | Mortality rate (%) |
| | 1 | 2 | 3 | 4 | 5 | 6 | 7 | 8 | 9 | 10 | |
| 6×10⁸ | 0/10 | 0/10 | 0/10 | 2/4 | 4/6 | 5/5 | 5/5 | 5/5 | 5/5 | 5/5 | 50 |
| 7×10⁸ | 0/10 | 0/10 | 1/9 | 2/4 | 3/7 | 5/5 | 6/4 | 7/3 | 8/2 | 8/2 | 80 |
| 8×10⁸ | 0/10 | 0/10 | 2/8 | 3/7 | 5/5 | 6/4 | 8/2 | 9/1 | 10/0 | 10/0 | 100 |
| 9×10⁸ | 0/10 | 0/10 | 2/8 | 3/7 | 6/4 | 8/2 | 9/1 | 10/0 | 10/0 | 10/0 | 100 |
| Intact mice | 0/10 | 0/10 | 0/10 | 0/10 | 0/10 | 0/10 | 0/10 | 0/10 | 0/10 | 0/10 | 0 |

LD₁₀₀St.a. (MRSA) 8×10⁸ (the minimum dose of St.a. providing 100% mortality rate among mice) was used in the main experiment.

### Determination of ED of ampicillin on the model of staphylococcal sepsis in mice

As follows from the data given in Table 17, the first fatal case was registered on day 2 after infection with a lethal staphylococcus dose. The first fatal case in the tested groups was registered on the second day in a dose of 30 mg/kg, on the third day in doses of 60, 90, 120 mg/kg, and on the fourth day in doses of 150 and 180 mg/kg. The last mouse in the control group died in the experiment on day 7. On day 10, the mortality rate was the following: 0 in doses 30 and 60 mg/kg, 10% in a dose of 90 mg/kg, and 20% in doses of 120, 150, and 180 mg/kg. The control (intact) group had no fatal cases.

**Table 17**

| Determination of "ED" of ampicillin on a model of staphylococcal sepsis in mice | | | | | | | | | | | |
|---|---|---|---|---|---|---|---|---|---|---|---|
| Doses Of ampicillin (+LD₁₀₀St.a.) | Days of experiment (died/survived animals) | | | | | | | | | | Survival rate (%) |
| | 1 | 2 | 3 | 4 | 5 | 6 | 7 | 8 | 9 | 10 | |
| 30 mg/kg | 0/10 | 1/9 | 3/7 | 6/4 | 8/2 | 9/1 | 10/0 | 10/0 | 10/0 | 10/0 | 0 |
| 60 mg/kg | 0/10 | 0/10 | 2/8 | 4/6 | 6/4 | 8/2 | 9/1 | 10/0 | 10/0 | 10/0 | 0 |
| 90 mg/kg | 0/10 | 0/10 | 1/9 | 3/7 | 5/5 | 7/3 | 8/2 | 9/1 | 9/1 | 9/1 | 10 |
| 120 mg/kg | 0/10 | 0/10 | 1/9 | 4/6 | 6/4 | 7/3 | 8/2 | 8/2 | 8/2 | 8/2 | 20 |
| 150 mg/kg | 0/10 | 0/10 | 0/10 | 3/7 | 5/5 | 7/3 | 7/3 | 8/2 | 8/2 | 8/2 | 20 |
| 180 mg/kg | 0/10 | 0/10 | 0/10 | 2/8 | 4/6 | 6/4 | 7/3 | 7/3 | 8/2 | 8/2 | 20 |
| 0 mg/kg | 0/10 | 0/10 | 3/7 | 5/5 | 6/4 | 8/2 | 9/1 | 10/0 | - | - | 0 |

ED₅₀ of ampicillin against the strain MRSA could not be determined since the survival rate in all groups was not more than 50%.

### Evaluation of the activity of glutaryl histamine in combination with ampicillin on the model of staphylococcal sepsis in mice

**Table 18**

| Determination of activity of glutaryl histamine in combination with ampicillin on an MRSA-induced model of staphylococcal sepsis in mice | | | | | | | | | | | | |
|---|---|---|---|---|---|---|---|---|---|---|---|---|
| Group No | Days of experiment (numerator -died animals, denominator - survived animals) | | | | | | | | | | Mortality rate, % | Survival rate, % |
| | 1 | 2 | 3 | 4 | 5 | 6 | 7 | 8 | 9 | 10 | | |
| 1 | 0/10 | 0/10 | 1/9 | 3/7 | 5/5 | 7/3 | 8/2 | 9/1 | 10/0 | 10/0 | 100 | 0 |
| 2 | 0/10 | 0/10 | 1/9 | 2/8 | 3/7 | 5/5 | 6/4 | 7/3 | 8/2 | 8/2 | 80 | 20 |
| 3 | 0/10 | 0/10 | 0/10 | 1/9 | 2/8 | 3/7 | 4/6 | 5/5 | 5/5 | 5/5 | 50 | 50 |
| 4 | 0/10 | 0/10 | 0/10 | 0/10 | 1/9 | 2/8 | 4/6 | 5/5 | 6/4 | 6/4 | 60 | 40 |
| 5 | 0/10 | 0/10 | 0/10 | 0/10 | 0/10 | 1/9 | 3/7 | 4/6 | 4/6 | 5/5 | 50 | 50 |
| 6 | 0/10 | 0/10 | 0/10 | 0/10 | 1/9 | 2/8 | 3/7 | 6/4 | 5/5 | 5/5 | 50 | 50 |

| | | | | | | | | | | | | |
|---|---|---|---|---|---|---|---|---|---|---|---|---|
| Groups for Table 18 1. Control, lethal dose of staphylococcus MSRA (8×10⁸, i.v. one time) 2. Staphylococcus (8×10⁸, i.v., one time)+ ampicillin (120 mg/ml, i.v., one time) 3. Staphylococcus (8×10⁸, i.v., one time)+ levofloxacin (4 mg/ml, *per os*, one time) 4. Staphylococcus (8×10⁸, i.v., one time)+ glutaryl histamine (15 mg/ml, *per os*, 10 days) + ampicillin (120 mg/ml, i.v., one time) 5. Staphylococcus (8×10⁸, i.v., one time)+ glutaryl histamine (30 mg/ml, *per os*, 10 days) + ampicillin (120 mg/ml, i.v., one time) 6. Staphylococcus (8×10⁸, i.v., one time) + glutaryl histamine (45 mg/ml, *per os*, 10 days) + ampicillin(120 mg/ml, i.v., one time). | | | | | | | | | | | | |

The analysis of the obtained results allows the following conclusion:
The use of the strain MRSA as an infectious agent provided the maximum survival rate of 20% (in a dose of ampicillin of 120 mg/kg).

A combination of ampicillin in adose of 120 mg/kg with glutaryl histamine in doses 15, 30 and 45 increased the survival rate to 40-50% compared with the control group with 100% mortality.

Combined administration of ampicillin with glutaryl histamine provides an enhanced effectiveness of the therapy (according to % of survived mice).

### Example 9. Potentiating the effectiveness of antibacterial therapy of pneumonia in combined administration of antibiotics and glutaryl histamine

Example 9.1. A patient with a diagnosis of double-sided multisegmental pneumonia was treated. When entered a hospital, the patient had temperature of 38.9°C and was in a moderate-to-severe condition. He had the marked symptoms such as pale face skin and cyanosis of the lips. Other observed symptoms were scleral injection and conjunctival hyperemia; hyperemic oropharyngeal mucous membrane, cyanosis and granulation in the mucous membrane in the soft palate. Dry cough that was painful in the tracheal area, respiratory rate was 20 breaths/min. Breath sounds in the lung were diminished with moist rales. The level of blood saturation was 96%. Heart sounds were muffled, rhythmic, and pulse rate was 100 beats/min. Blood pressure was 105/60 mmHg.

Chest X-ray showed intensive shadow with distinct boundaries in the right lobe (S7 and S5), caused by infiltration and interlobar effusion, and right-sided pleural reaction. In the left side (S9), there are focally confluent shadows against the background of enhanced vascular and interstitial pattern. Roots were dilated and structural. Conclusion: double sided multisegmental pleuropneumonia.

Admission general blood test: Leukocytes - 8.2^{∗}10⁹ g/l, platelets - 132^{∗}10⁹ g/l. Neutrophils: stab - 19%, segmented - 59%; lymphocytes - 17%; monocytes - 5%; ESR - 25 mm/h.

C-reactive protein (CRP) was 96 mg/l, and procalcitonin was 0.505 ng/ml.

The treatment was conducted according to the following scheme: glutaryl histamine 90 mg *per os* once a day for 5 days; cefotaxime 2.0 g, IM, three times a day for 8 days.

On the treatment with glutaryl histamine and antibiotic (cefotaxime) patient's condition improved, temperature reached the norm on day 3 (fifth day of the disease), the symptoms of headache, dizziness, and fatigue subsided at the same time. Cough began to decrease significantly from day 4 of the disease, infrequent hacking cough and changes in auscultation of the lungs (single moist rales) continued to persist up to day 9.

Chest X-ray 10 days after admission showed only local prominence and deformation of the lung pattern in the right lower lobe. In the left side, the lung field was completely clear.

The control blood test did not reveal abnormalities, the values of CRP and procalcitonin after the treatment completely normalized.

General blood test 8 days after admission: Leukocytes - 3.8^{∗}10⁹ g/l, platelets - 37^{∗}10⁹ g/l. Neutrophils: stab - 2%, segmented - 49%; lymphocytes - 37%; monocytes - 10%; eosinophils- 2%; ESR - 10 mm/h; CRP - negative; and procalcitonin - 0.035 hg/ml.

This clinical example demonstrates successful treatment of pneumonia with markers of active bacterial infection: neutrophil-stab shift in the peripheral blood, high levels of C-reactive protein and procalcitonin >0.05.

Example 9.2. A patient with a diagnosis of right-focal confluent pneumonia was treated. When entered a hospital, the patient was in a moderate-to-severe condition. The patient had pale skin, moderate cyanosis of the lips, scleral injection and conjunctival hyperemia; rough nasal breathing; hyperemic oropharyngeal mucosa membrane with cyanosis in the soft palate, and granulation in the mucous membrane on the posterior wall. Voice was hoarse. Cough was dry, painful and attack-like. Admission chest X-ray showed an intensification of the lung pattern in the right basal segments of the lower lobe, against which there were focally confluent shadows of weak intensity caused by pneumonic infiltration. The right root was dilated. Conclusion: right-focal confluent pneumonia.

Admission general blood test: Leukocytes - 9.8^{∗}10⁹ g/l, platelets - 174^{∗}10⁹ g/l; Neutrophils: stab - 6%, segmented - 72%; lymphocytes - 10%; monocytes - 12%; ESR - 2 mm/h. CRP - 96 mg/l.

The treatment was conducted according to the following scheme: glutaryl histamine 90 mg *per os* once a day for 5 days; cefotaxim 2.0 g, IM, three times a day for 10 days; macropene (midecamycin) - 400 mg *per os* twice a day for 10 days.

The course of the disease was favorable; the patient was in a satisfactory condition on day 4. Temperature was steadily normal from the third day of the disease, x-ray of the chest and blood parameters reached normal condition to day 12. Chest X-ray a week after admission showed only local prominence and deformation of the lung pattern in the right lower lobe. The bronchial vascular pattern in the rest segments was intensive. Roots were not dilated.

General blood test 7 days after admission: Leukocytes - 6.6^{∗}10⁹ g/l, platelets - 260^{∗}10⁹ g/l; Neutrophils: segmented - 73%; lymphocytes - 20%; monocytes - 4%; eosinophils - 3%; ESR - 4 mm/h; CRP - negative.

This clinical example demonstrates successful treatment of pneumonia treated according to the present invention. Administration of the combined therapy according to the invention allowed the optimal clinical effect.

### Example 9.3. Right-sided multisegmental pneumonia

When entered a hospital, the patient had temperature of 39.5°C, and was in a moderate-to-severe condition. Clinical examination revealed the following symptoms: skin pallor, cyanosis of the lips, scleral injection, conjunctival hyperemia; diffusely hyperemic oropharyngeal mucosa membrane, granulation in the soft palate, hypertrophic follicles on the posterior wall. Nasal breathing was rough due to stuffiness in nose. Cough was chesty. Respiratory rate was 20 breaths/min. In the right part of the lungs, breath sounds were diminished with moist and dry rales, but in the left side breathing was harsh. The level of blood saturation was 90%. Heart sounds were muffled, rhythmic, and pulse rate was 90 beats/min; blood pressure was 110/70 mmHg. Admission chest X-ray showed a shadow with distinct boundaries in segment S6 of the right lower lobe and in the upper lobe (S1). Right-sided pleural reaction was seen. Roots were dilated and structural. Conclusion: right-sided multisegmental pneumonia.

Admission general blood test: Leukocytes - 7.1^{∗}10⁹ g/l, platelets - 109^{∗}10⁹ g/l. Neutrophils: stab - 19%, segmented - 55%; lymphocytes - 17%; monocytes - 9%; toxic granulosity ++; ESR - 40 mm/h; CRP - 192 mg/l; and procalcitonin - 15.8 hg/ml.

The treatment was conducted according to the following scheme: glutaryl histamine 90 mg *per os* once a day for 5 days; cefotaxime - 2.0 g IM three times a day for 10 days; azithromycin - 500.0 once a day for 3 days. The therapy was symptomatic.

The course of the disease was favorable; temperature was normal from the fifth day of the disease. The patient was in a satisfactory condition from day 8. Chest X-ray 10 days after admission showed that the lung field in segment S6 was completely clear. The pattern in the left upper lobe was deformed, interlobar pleura was tucked, and in the apex there were pleural deposits.

General blood test 10 days after admission: Leukocytes - 7.1^{∗}10⁹ g/l, platelets - 275^{∗}10⁹ g/l. Neutrophils: stab - 2%, segmented - 44%; lymphocytes - 36%; monocytes - 16%; eosinophils - 2%; ESR - 24 mm/h; CRP - negative; and procalcitonin - 0.193 hg/ml.

This clinical example demonstrates successful treatment of pneumonia. Bacterial nature of pneumonia followed from the following laboratory data: sharp neutrophil shift, toxic granulosity, high levels of procalcitonin and C-reactive protein. Combined therapy with glutaryl histamine and antibiotics provides better clinical effect characterized by fast (on day 13 of the disease, day 10 after the treatment) normalization of the blood condition, inflammation markers and x-ray image.

Example 9.4. A patient with a diagnosis of right bilobar pneumonia was treated. When entered a hospital, the patient had temperature of 38.4°C, and was in a moderate-to-severe condition. On this day he started coughing. Clinical examination revealed the following symptoms: skin color was normal, and cyanosis was absent. There were scleral injection, conjunctival hyperemia; marked granulation in the soft palate, hypertrophic follicles on the posterior wall. Nose was stuffed, without discharge. Cough was soft non-productive. Breathing was harsh. The level of blood saturation was 97%. Heart sounds were rhythmic and muffled; pulse rate was 96 beats/min.

Admission chest X-ray (conclusion): right bilobar pneumonia.

Admission general blood test: Leukocytes - 7.9^{∗}10⁹ g/l, platelets - 163^{∗}10⁹ g/l. Neutrophils: stab - 2%, segmented - 70%; lymphocytes - 22%; monocytes - 5%; eosinophils - 1%; ESR - 31 mm/h; CRP - 192 mg/l; and procalcitonin - 0.124 hg/ml.

Admission sputum had a growth of *Str. viridans*× 10⁷, *Hemophylus sp* × 10⁷.

The treatment was conducted according to the following scheme: glutaryl histamine 90 mg *per os* once a day for 5 days; azithromycin 0.5 g once a day for 3 days; cefasoline 2 g three times a day for 12 days.

On the treatment, temperature was normal from the eight day of the disease (day 5 of the treatment). Cough decreased, but infrequent hacking continued up to day 9 of the disease.

Chest X-ray 16 days after admission did not show pathological and infiltrative tissues. The lung pattern was not changed. Roots were not dilated.

General blood test 8 days after the treatment: Leukocytes - 5.9^{∗}10⁹ g/l, platelets - 451^{∗}10⁹ g/l. Neutrophils: stab - 1%, segmented - 58%; lymphocytes - 35%; monocytes - 6%; eosinophils - 0; ESR - 10 mm/h; CRP - 6 mg/l; and procalcitonin - 0.033 hg/ml.

This example demonstrates successful treatment of the patient with bacterial pneumonia supported by the markers of bacterial infection. On day 10, condition of blood normalized, the levels of procalcitonin and CRP reached the norm values. Pneumonic infiltration in the lungs was significantly decreased at the same time.

Given the above clinical examples, the introduction of glutaryl histamine into combined therapy of pneumonia provides the effectiveness of the treatment, which is confirmed by not only early achievement of clinic stabilization but also by faster ingression of radiological changes in the lungs (Pneumonia, ed. by A.G. Chuchalin and et al., Moscow, 2002, pp.366-368 (480)).

### Example 10. Dosage forms of glutaryl histamine

For the treatment of the above-indicated diseases, glutaryl histamine can be administered orally, intramuscularly or intravenously in the unit dosage forms comprising non-toxic pharmaceutically acceptable carriers.

Glutaryl histamine may be administered to a patient in daily dose of from 0.1 to 100 mg/kg of human body weight, preferably in a dose of from 0.5 to 50 mg/kg, one or more times per day.

It must be noted that a particular dose for a particular patient is depend on many factors, such as patient's age, body weight, gender, general health condition, and diet; the schedule and route of the agent administration and the excretion rate of the agent from the body; a specific combination of medicaments and severity of a disease in a subject to be treated.

The pharmaceutical compositions according to the invention comprise glutaryl histamine in an amount efficient for providing a desired result, and may be administered in the form of a unit dosage form (for example, in solid, semi-solid, or liquid form) that comprises glutaryl histamine as an active agent in a mixture with a carrier or an excipient suitable for intravenous and oral administration. The active agent may be included into the composition together with conventional nontoxic pharmaceutically acceptable carriers suitable for the manufacture of solutions, tablets, pills, capsules, pellets, and any other dosage forms.

Various excipients may be used, such as saccharides, for example, glucose, lactose, of sucrose; mannitol or sorbitol; cellulose derivatives; and/or calcium phosphates, for example, tricalcium phosphate or calcium hydrogen phosphate. The following binders may be used: starch paste (for example, corn, wheat, rice, or potato starch), gelatin, tragacanth, methylcellulose, hydroxypropyl methylcellulose, sodium carboxymethyl cellulose, and/or polyvinylpyrrolidone. If necessary, disintegrating agents may be used, such as the aforementioned starches and carboxymethyl starch, crosslinkedpolyvinylpyrrolidone, agar-agar, or alginic acid or a salt thereof, such as sodium alginate.

Optional additives may be used, such as flowability control agents and lubricants, such as silica, talc, stearic acid and salts thereof, for example, magnesium stearate or calcium stearate, and/or propylene glycol.

Stabilizing, thickening, colorant, and fragrance additives may also be used.

In preparing a unit dosage form, the amount of an active agent used in combination with a carrier may vary depending on the recipient under the therapy and on the route of administration of therapeutic agent.

For example, when glutaryl histamine is used in the form of a solution for injection, the active agent in this solution is in an amount of 0.01 to 5 wt.%. As diluents the following solutions are suitable: 0.9% sodium chloride solution, distilled water, Novocaine solution for injections, Ringer solution, glucose solution, and specific solubilizing adjuvants. When glutaryl histamine is administered in the form of tablets, its amount is 5.0 to 500 mg per unit dosage form.

Dosage forms of glutaryl histamine used according to the present invention are prepared by the standard methods such as, for example, processes of mixing, granulating, forming pills, dissolving and lyophilizing.

### Tableted form

A tableted form is prepared by using the following ingredients:

| | |
|---|---|
| Glutaryl histamine or a pharmaceutically acceptable salt thereof | 1-100 mg |
| Potato starch | 20-50 mg |
| Magnesium stearate | 3 mg |
| Aerosil | 1 mg |
| Lactose | up to 300 mg |
| The ingredients are mixed and compressed to form tablets weighing | 300 mg. |

### Suppositories

Example of a suppository formulation

| | |
|---|---|
| Glutaryl histamine or a pharmaceutically acceptable salt thereof | 1-100 mg |
| Cacao oil | in an amount required for a suppository |

If necessary, a rectal, vaginal, and urethral suppository can be prepared with corresponding excipients.

### Dry powder for inhalation

Example of a powder formulation:

| | |
|---|---|
| Glutaryl histamine or a pharmaceutically acceptable salt thereof | 0.2 g |
| Lactose | up to 1 g |

A powder is placed in a special device (container) or a gelatin capsule

### Nasal spray

Example of a spray formulation:

| | |
|---|---|
| Glutaryl histamine or a pharmaceutically acceptable salt thereof | 1.5-150 mg |
| Purified water | up to 15 ml |

### Solution for injection

Example of the formulation of a solution for injections:

| | |
|---|---|
| Glutaryl histamine or a pharmaceutically acceptable salt thereof | 1-50 mg |
| Water for injection | 2 ml |

## Claims

1. A medicament or a pharmaceutical composition for use in the treatment of respiratory tract disease selected from the group consisting of sinusitis, tonsillitis, and acute respiratory distress syndrome, wherein
the medicament comprises glutaryl histamine or a pharmaceutically acceptable salt thereof in an effective amount, and
the pharmaceutical composition comprises glutaryl histamine or a pharmaceutically acceptable salt thereof in an effective amount, and a pharmaceutically acceptable carrier or diluent.

2. A medicament comprising glutaryl histamine or a pharmaceutically acceptable salt thereof in an effective amount, or a pharmaceutical composition comprising glutaryl histamine or a pharmaceutically acceptable salt thereof in an effective amount, and a pharmaceutically acceptable carrier or diluent for use in potentiating the effectiveness of antibacterial therapy in the treatment of respiratory tract diseases.

3. The medicament or the pharmaceutical composition for use according to claim 2, wherein the antibacterial therapy comprises administering of at least one of one antibacterial drug selected from the group including cefotaxime, midecamycin, azithromycin, amoxicillin, levofloxacin, oxacillin, vancomycin, and ceftriaxone.

4. The medicament or the pharmaceutical composition for use according to claim 3, wherein the antibacterial therapy is targeted to microorganisms with a reduced sensitivity to antibacterial drugs or to microorganisms resistant to antibacterial therapy.

5. The medicament or the pharmaceutical composition for use according to claim 4, wherein said microorganisms include at least one species selected from *Staphylococcus aureus, Streptococcus pneumonia, Haemophylus influenza,* and *Moraxella catarrhalis.*

6. The medicament or the pharmaceutical composition for use according to any of claims 1 to 5, wherein the composition or the medicament is administered in an amount providing a dose of glutaryl histamine or a pharmaceutically acceptable salt thereof of 0.1 to 100 mg/kg of body weight.

7. The medicament or the pharmaceutical composition for use according to claim 6, wherein the composition or the medicament is administered in an amount providing a dose of glutaryl histamine or a pharmaceutically acceptable salt thereof of 0.5 to 5 mg/kg of body weight.

8. The medicament or the pharmaceutical composition for use according to any of claims 1 to 5, wherein the administration is an oral administration or a daily administration.

9. The medicament or the pharmaceutical composition for use according to any one of claims 2 to 5, wherein the respiratory tract disease is selected from the group consisting of tonsillitis and pneumonia.

10. Glutaryl histamine or a pharmaceutically acceptable salt thereof for use in the treatment of a respiratory tract disease selected from the group consisting of sinusitis, tonsillitis, and acute respiratory distress syndrome.

11. Glutaryl histamine or a pharmaceutically acceptable salt thereof for use in potentiating the effectiveness of antibacterial therapy in the treatment of respiratory tract diseases.

12. Glutaryl histamine or a pharmaceutically acceptable salt thereof for use according to claim 11, wherein the antibacterial therapy comprises administering of at least one antibacterial drug selected from the group including cefotaxime, midecamycin, azithromycin, amoxicillin, levofloxacin, oxacillin, vancomycin, and ceftriaxone.

13. Glutaryl histamine or a pharmaceutically acceptable salt thereof for use according to claim 12 wherein the antibacterial therapy is targeted to microorganisms with a reduced sensitivity to antibacterial drugs or to microorganisms resistant to antibacterial therapy.

14. Glutaryl histamine or a pharmaceutically acceptable salt thereof for use according to claim 13, wherein said microorganisms include at least one species selected from *Staphylococcus aureus, Streptococcus pneumonia, Haemophylus influenza,* and *Moraxella catarrhalis.*

15. Glutaryl histamine or a pharmaceutically acceptable salt thereof for use according to any of claims 10 to 14, wherein the dose of glutaryl histamine or a pharmaceutically acceptable salt thereof is of 0.1 to 100 mg/kg of body weight.

16. Glutaryl histamine or a pharmaceutically acceptable salt thereof for use according to claim 15, wherein the dose of glutaryl histamine or a pharmaceutically acceptable salt thereof is of 0.5 to 5 mg/kg of body weight.

17. Glutaryl histamine or a pharmaceutically acceptable salt thereof for use in the treatment of sepsis.

## Patentansprüche

1. Medikament oder pharmazeutische Zusammensetzung zur Verwendung bei der Behandlung von Atemwegserkrankungen ausgewählt aus der Gruppe bestehend aus Sinusitis, Tonsillitis und Atemnotsyndrom, wobei
das Medikament Glutarylhistamin oder ein pharmazeutisch akzeptables Salz davon in einer wirksamen Menge umfasst, und
die pharmazeutische Zusammensetzung Glutarylhistamin oder ein pharmazeutisch akzeptables Salz davon in einer wirksamen Menge und einen pharmazeutisch akzeptablen Träger oder Verdünner umfasst.

2. Medikament, das Glutarylhistamin oder ein pharmazeutisch akzeptables Salz davon in einer wirksamen Menge umfasst, oder pharmazeutische Zusammensetzung, die Glutarylhistamin oder ein pharmazeutisch akzeptables Salz davon in einer wirksamen Menge und einen pharmazeutisch akzeptablen Träger oder Verdünner umfasst, zur Verwendung bei der Verstärkung der Wirksamkeit einer antibakteriellen Therapie bei der Behandlung von Atemwegserkrankungen.

3. Medikament oder pharmazeutische Zusammensetzung zur Verwendung gemäß Anspruch 2, wobei die antibakterielle Therapie es umfasst, dass mindestens ein antibakterielles Arzneimittel ausgewählt aus der Gruppe, die Cefotaxim, Midecamycin, Azithromycin, Amoxicillin, Levofloxacin, Oxacillin, Vancomycin und Ceftriaxon einschließt, verabreicht wird.

4. Medikament oder pharmazeutische Zusammensetzung zur Verwendung gemäß Anspruch 3, wobei die antibakterielle Therapie auf Mikroorganismen mit einer verringerten Sensibilität gegenüber antibakteriellen Arzneimitteln oder auf Mikroorganismen, die gegenüber antibakterieller Therapie resistent sind, gerichtet ist.

5. Medikament oder pharmazeutische Zusammensetzung zur Verwendung gemäß Anspruch 4, wobei die Mikroorganismen mindestens eine Spezies, ausgewählt aus *Staphylococcus aureus, Streptococcus pneumonia, Haemophylus influenza* und *Moraxella catarrhalis,* einschließen.

6. Medikament oder pharmazeutische Zusammensetzung zur Verwendung gemäß mindestens einem der Ansprüche 1 bis 5, wobei die Zusammensetzung oder das Medikament in einer Menge, die eine Dosis an Glutarylhistamin oder einem pharmazeutisch akzeptablen Salz davon von 0,1 bis 100 mg/kg Körpergewicht bereitstellt, verabreicht wird.

7. Medikament oder pharmazeutische Zusammensetzung zur Verwendung gemäß Anspruch 6, wobei die Zusammensetzung oder das Medikament in einer Menge, die eine Dosis an Glutarylhistamin oder einem pharmazeutisch akzeptablen Salz davon von 0,5 bis 5 mg/kg Körpergewicht bereitstellt, verabreicht wird.

8. Medikament oder pharmazeutische Zusammensetzung zur Verwendung gemäß mindestens einem der Ansprüche 1 bis 5, wobei die Verabreichung eine orale Verabreichung oder eine tägliche Verabreichung ist.

9. Medikament oder pharmazeutische Zusammensetzung zur Verwendung gemäß mindestens einem der Ansprüche 2 bis 5, wobei die Atemwegserkrankung ausgewählt ist aus der Gruppe bestehend aus Tonsillitis und Lungenentzündung.

10. Glutarylhistamin oder pharmazeutisch akzeptables Salz davon zur Verwendung bei der Behandlung von Atemwegserkrankungen ausgewählt aus der Gruppe bestehend aus Sinusitis, Tonsillitis und Atemnotsyndrom.

11. Glutarylhistamin oder pharmazeutisch akzeptables Salz davon zur Verwendung bei der Verstärkung der Wirksamkeit einer antibakteriellen Therapie bei der Behandlung von Atemwegserkrankungen.

12. Glutarylhistamin oder pharmazeutisch akzeptables Salz davon zur Verwendung gemäß Anspruch 11, wobei die antibakterielle Therapie es umfasst, dass mindestens ein antibakterielles Arzneimittel ausgewählt aus der Gruppe, die Cefotaxim, Midecamycin, Azithromycin, Amoxicillin, Levofloxacin, Oxacillin, Vancomycin und Ceftriaxon einschließt, verabreicht wird.

13. Glutarylhistamin oder pharmazeutisch akzeptables Salz davon zur Verwendung gemäß Anspruch 12, wobei die antibakterielle Therapie auf Mikroorganismen mit einer verringerten Sensibilität gegenüber antibakteriellen Arzneimitteln oder auf Mikroorganismen, die gegenüber antibakterieller Therapie resistent sind, gerichtet ist.

14. Glutarylhistamin oder pharmazeutisch akzeptables Salz davon zur Verwendung gemäß Anspruch 13, wobei die Mikroorganismen mindestens eine Spezies, ausgewählt aus *Staphylococcus aureus, Streptococcus pneumonia, Haemophylus influenza* und *Moraxella catarrhalis,* einschließen.

15. Glutarylhistamin oder pharmazeutisch akzeptables Salz davon zur Verwendung gemäß mindestens einem der Ansprüche 10 bis 14, wobei die Dosis an Glutarylhistamin oder einem pharmazeutisch akzeptablen Salz davon 0,1 bis 100 mg/kg Körpergewicht beträgt.

16. Glutarylhistamin oder pharmazeutisch akzeptables Salz davon zur Verwendung gemäß Anspruch 15, wobei die Dosis an Glutarylhistamin oder einem pharmazeutisch akzeptablen Salz davon von 0,5 bis 5 mg/kg Körpergewicht beträgt.

17. Glutarylhistamin oder pharmazeutisch akzeptables Salz davon zur Verwendung bei der Behandlung von Sepsis.

## Revendications

1. Médicament ou composition pharmaceutique pour son utilisation dans le traitement de maladies des voies respiratoires sélectionnées à partir du groupe constitué par la sinusite, l'amygdalite, et le syndrome de détresse respiratoire aiguë, dans lequel
le médicament comprend de la glutaryl histamine ou un sel pharmaceutiquement acceptable de celle-ci en une quantité efficace, et
la composition pharmaceutique comprend de la glutaryl histamine ou un sel pharmaceutiquement acceptable de celle-ci en une quantité efficace, et un véhicule ou un diluant pharmaceutiquement acceptables.

2. Médicament comprenant de la glutaryl histamine ou un sel pharmaceutiquement acceptable de celle-ci en une quantité efficace, ou une composition pharmaceutique comprenant de la glutaryl histamine ou un sel pharmaceutiquement acceptable de celle-ci en une quantité efficace, et un véhicule ou diluant pharmaceutiquement acceptables pour son utilisation dans la potentialisation de l'efficacité d'une thérapie antibactérienne dans le traitement de maladies des voies respiratoires.

3. Médicament ou composition pharmaceutique pour son utilisation selon la revendication 2, dans lequel la thérapie antibactérienne comprend l'administration d'au moins une substance pharmaceutique antibactérienne sélectionnée à partir du groupe incluant le céfotaxime, la midécamycine, l'azithromycine, l'amoxicilline, la lévofloxacine, l'oxacilline, la vancomycine, et la ceftriaxone.

4. Médicament ou composition pharmaceutique pour son utilisation selon la revendication 3, dans lequel la thérapie antibactérienne cible les microorganismes avec une sensibilité réduite aux substances pharmaceutiques antibactériennes ou les microorganismes résistants à une thérapie antibactérienne.

5. Médicament ou composition pharmaceutique pour son utilisation selon la revendication 4, dans lequel lesdits microorganismes incluent au moins une espèce sélectionnée à partir de *Staphylococcus aureus, Streptococcus pneumonia, Haemophylus influenza,* et *Moraxella catarrhalis.*

6. Médicament ou composition pharmaceutique pour son utilisation selon l'une quelconque des revendications 1 à 5, dans lequel la composition ou le médicament est administré en une quantité fournissant une dose de glutaryl histamine ou d'un sel pharmaceutiquement acceptable de celle-ci de 0,1 à 100 mg/kg de poids corporel.

7. Médicament ou composition pharmaceutique pour son utilisation selon la revendication 6, dans lequel la composition ou le médicament est administré en une quantité fournissant une dose de glutaryl histamine ou d'un sel pharmaceutiquement acceptable de celle-ci de 0,5 à 5 mg/kg de poids corporel.

8. Médicament ou composition pharmaceutique pour son utilisation selon l'une quelconque des revendications 1 à 5, dans lequel l'administration est une administration orale ou une administration quotidienne.

9. Médicament ou composition pharmaceutique pour son utilisation selon l'une quelconque des revendications 2 à 5, dans lequel la maladie des voies respiratoires est sélectionnée à partir du groupe constitué par l'amygdalite et la pneumonie.

10. Glutaryl histamine ou sel pharmaceutiquement acceptable de celle-ci pour son utilisation dans le traitement d'une maladie des voies respiratoires sélectionnée à partir du groupe constitué par la sinusite, l'amygdalite, et le syndrome de détresse respiratoire aiguë.

11. Glutaryl histamine ou sel pharmaceutiquement acceptable de celle-ci pour son utilisation dans la potentialisation de l'efficacité d'une thérapie antibactérienne pour le traitement de maladies des voies respiratoires.

12. Glutaryl histamine ou sel pharmaceutiquement acceptable de celle-ci pour son utilisation selon la revendication 11, dans laquelle la thérapie antibactérienne comprend l'administration d'au moins une substance pharmaceutique antibactérienne sélectionnée à partir du groupe incluant le céfotaxime, la midécamycine, l'azithromycine, l'amoxicilline, la lévofloxacine, l'oxacilline, la vancomycine, et la ceftriaxone.

13. Glutaryl histamine ou sel pharmaceutiquement acceptable de celle-ci pour son utilisation selon la revendication 12 dans laquelle la thérapie antibactérienne cible les microorganismes avec une sensibilité réduite aux substances pharmaceutiques antibactériennes ou les microorganismes résistants à une thérapie antibactérienne.

14. Glutaryl histamine ou sel pharmaceutiquement acceptable de celle-ci pour son utilisation selon la revendication 13, dans laquelle lesdits microorganismes incluent au moins une espèce sélectionnée à partir de *Staphylococcus aureus, Streptococcus pneumonia, Haemophylus influenza,* et *Moraxella catarrhalis.*

15. Glutaryl histamine ou sel pharmaceutiquement acceptable de celle-ci pour son utilisation selon l'une quelconque des revendications 10 à 14, dans laquelle la dose de glutaryl histamine ou d'un sel pharmaceutiquement acceptable de celle-ci est de 0,1 à 100 mg/kg de poids corporel.

16. Glutaryl histamine ou sel pharmaceutiquement acceptable de celle-ci pour son utilisation selon la revendication 15, dans laquelle la dose de glutaryl histamine ou d'un sel pharmaceutiquement acceptable de celle-ci est de 0,5 à 5 mg/kg de poids corporel.

17. Glutaryl histamine ou sel pharmaceutiquement acceptable de celle-ci pour son utilisation dans le traitement du sepsis.
